# EUROPEAN PATENT SPECIFICATION

(11) **EP 1 450 855 B1**
(45) Date of publication and mention of the grant of the patent: **26.08.2009**
(21) Application number: 02751507.1
(22) Date of filing: 23.05.2002
(51) Int. Cl.: A61K 39/39, A61K 39/35, A61K 39/106, A61K 39/108, A61P 37/00

(54) **SUPPRESSION OF ALLERGIC REACTIONS BY TRANSDERMAL ADMINISTRATION OF ALLERGENS IN CONJUNCTION WITH OR CONJUGATED TO TOXIN SUBUNITS OR FRAGMENTS THEREOF**
UNTERDRÜCKUNG VON ALLERGISCHEN REAKTIONEN DURCH TRANSKUTANE VERABREICHUNG VON ALLERGENEN ZUSAMMEN MIT ODER FUSIONIERT MIT TOXINUNTEREINHEITEN ODER DEREN FRAGMENTEN
SUPPRESSION DE REACTIONS ALLERGIQUES PAR ADMINISTRATION INTRADERMIQUE D'ALLERGENES CONJUGUES AVEC DES SOUS UNITES DE TOXINE OU DES FRAGMENTS DE CELLE-CI

(30) Priority: 23.05.2001 US 293142 P
(43) Date of publication of application: 01.09.2004
(73) Proprietor: Duotol AB, 426 74 Västra Frölunda (SE)
(72) Inventor: HOLMGREN, Jan, 426 74 Vastra Frolunda (SE); CZERKINSKY, Cecil, 06300 Nice (FR)
(74) Representative: Bernasconi, Jean Raymond
(86) International application number: PCT/IB2002/003053
(87) International publication number: WO 2002/093998

(56) References cited:
- WO-A-98/20734
- WO-A-98/47529
- WO-A-99/43350
- WO-A-99/66947
- WIEDERMANN URSULA ET AL: "Suppressive versus stimulatory effects of allergen/cholera toxoid (CTB) conjugates depending on the nature of the allergen in a murine model of type I allergy." INTERNATIONAL IMMUNOLOGY, vol. 11, no. 7, July 1999 (1999-07), pages 1131-1138, XP002240878 ISSN: 0953-8178
- RASK C ET AL: "Prolonged oral treatment with low doses of allergen conjugated to cholera toxin B subunit suppresses immunoglobulin E antibody responses in sensitized mice." CLINICAL AND EXPERIMENTAL ALLERGY, vol. 30, no. 7, July 2000 (2000-07), pages 1024-1032, XP009010801 ISSN: 0954-7894
- GLENN G M ET AL: "Transcutaneous immunization with bacterial ADP-ribosylating exotoxins as antigens and adjuvants" INFECTION AND IMMUNITY, AMERICAN SOCIETY FOR MICROBIOLOGY. WASHINGTON, US, vol. 67, no. 3, March 1999 (1999-03), pages 1100-1106, XP002110054 ISSN: 0019-9567
- GLENN ET AL: "Advances in vaccine delivery: transcutaneous immunisation" EXPERT OPINION ON INVESTIGATIONAL DRUGS, ASHLEY PUBLICATIONS LTD., LONDON, GB, vol. 8, no. 6, June 1999 (1999-06), pages 797-805, XP002110056 ISSN: 1354-3784

## Description

### FIELD OF THE INVENTION

The present invention relates to a method for the suppression of type I hypersensitivity reactions in mammals by the transdermal administration of a specific allergen (or allergens) administered with or linked to the mucosal binding subunit of cholera toxin or fragments thereof.

### BACKGROUND OF THE INVENTION

### Allergy

Allergy is an ailment that affects millions of individuals worldwide. Attempts to desensitize an individual against a material that causes an allergic response (hereafter designated as an "allergen") by injection of measured dosages of the allergen heretofore has failed to achieve complete relief of allergy symptoms reproducibly in all allergic individuals. An allergic response is a term of art and has a well-defined meaning. Within the context of the present invention, an allergic or reagenic response includes, in particular, at least one of the features of 1.) production of an abnormally high level of gE in an individual's serum, 2.) immunologic interaction between an allergen, an individual's IgE and leukocytes resulting in release of histamines, 3.) production of hives, rashes, wheel and flare and similar dermatological manifestation of hypersensitivity and 4.) anaphylaxis.

An allergic response is a state of hypersensitivity in which an exaggerated immune response is induced by the exposure to a particular antigen or allergen. Hypersensitivity reactions can be classified as immediate or delayed. Immediate or type I hypersensitivity (or anaphylactic response) is a form of allergic reaction which develops very quickly, *i*.*e*., within seconds or minutes of exposure of the patient to the causative allergen, and it is mediated by IgE antibodies produced by B lymphocytes. In non-allergic patients, there is no IgE antibody of clinical relevance; but, in a person suffering with allergic diseases, IgE antibody mediates immediate hypersensitivity by sensitizing mast cells which are abundant in the skin, lymphoid organs, in the membranes of the eye, nose and mouth, and in the respiratory tree and intestines.

IgE secreted from activated B cells can attach to Fc receptors located on the surface of mast cells and basophil granulocytes, which contain numerous cytoplasmic granules packed with chemical mediators e.g. histamine (J. Klein, "Immunology", Blackwell Sci. Pub., London, 1990; E. Benjamini & S. Leskowitz, "Immunology", Wiley-Liss, N.Y. 1991). This receptor binds circulating IgE with very high affinity and retains it at the mast cell surface for extended periods of time. Activation is accomplished through the binding of an allergen simultaneously to more than one polyvalent molecule of Fc receptor-bound IgE. This "cross linking" of at least two surface-bound IgE molecules brings Fc receptor proteins into close association with one another in the plane of the mast cell plasma membrane. When the bound IgE is contacted by the appropriate allergen, the mast cell becomes activated. Kinases associated with these receptors become activated as a result of this proximity, initiating the second messenger cascade, which results in the fusion of the granules with the cell surface membrane, leading to the exocytosis of the granule contents, such as histamine, cytokines, and leukotrienes into the surrounding tissue, and the concomitant induction of allergic symptoms. It is the activity of these substances which is responsible for the clinical symptoms typical of immediate hypersensitivity such as contraction of smooth muscle in the airways or the intestine, the dilation of small blood vessels and the increase in their permeability to water and plasma proteins, the secretion of thick sticky mucus, and in the skin, redness, swelling and the stimulation of nerve endings that results in itching or pain.

Delayed type hypersensitivity (DTH) reactions are mediated by T-cells and macrophages and become evident only after 1 to 2 days and persist from several days to a few weeks. DTH is also referred to as cell-mediated hypersensitivity (*i*.*e*., T-cell mediated) and is part of a larger group of reactions called cell-mediated immunity.

Anaphylaxis, or anaphylactic shock is an acute systemic (whole body) type of allergic reaction. It occurs when a person has become sensitized to a certain substance or allergen (that is, the immune system has been abnormally triggered to recognize that allergen as a threat to the body). On the second or subsequent exposure to the substance, an allergic reaction occurs. This reaction is sudden, severe, and involves the whole body. Anaphylaxis is life-threatening and can occur at any time.

Therapeutically, many agents are used to try to prevent the release of mediators from mast cells and basophils and/or to treat the downstream events by blocking or ameliorating the effects of the mediators on target tissues. Therapeutic agents commonly employed fall under the following main groups:
1) Antihistamines block and mop up the released histamine, *i*.*e*. the major mediator of the allergic response.
2) β1 β2 agonist, *e*.*g*., Epinephrine, Salbutamol overcome indirectly the downstream effects on vasculature and smooth muscle.
3)Chromoglycate is useful for primary prevention of mast cells/basophil degranulation. This prophylactic must be taken continuously. It does not prevent the cross-linking of IgE but it somehow interferes with subsequent events.
4) Theophylline and other phosphodiesterase inhibitors again influence downstream biochemical events particularly associated with cyclic nucleotides.
5) Steroids have multiple sites of activities against the allergic response. They are either administered locally and/or systematicallysystemically.

None of the above treatments are ideal for the modulation of allergic responses because each has specific problems such as side effects including drowsiness, they also lack specificity in the immune system leading to global immunosuppression. Also many of these therapeutic agents need to be administered continuously. Therefore, new improved treatments are constantly being sought to control the allergic response prophylactically and/or therapeutically without the above-mentioned limitations.

Individuals who wish to become desensitized against an allergen often must submit himself/herself to injections of measured doses of the allergen, first administered at weekly or biweekly intervals, then gradually decreases to bimonthly or monthly intervals throughout the year. Such injections generally commence with a small dose of the allergen and then gradually increasing the dosage until a maximally-tolerated maintenance dose is achieved. The individual is then kept on a maintenance dose of the allergen for long periods of time or until the individual no longer exhibits an allergic reaction to the allergen.

Other treatment regimes have been devised to reduce or eliminate an allergic response.
Allergen injection therapy (allergen immunotherapy also known as subcutaneous immunotherapy (SCIT) is known to reduce the severity of allergic rhinitis. This treatment is theorized to involve the production of a different form of antibody, a protective antibody which is termed a "blocking antibody" (Cooke, RA et al., Serologic Evidence of Immunity with Coexisting Sensitization in a Type of Human Allergy, Exp. Med. 1935; 62:733). Chemical agents have been developed which inhibit the interactions between the IgE and its receptor (Cheng et al., U.S. Patent No. 5,965, 605 and Ra et al., U.S. Patent No. 6,090,034). IgE antagonists have also been used to treat allergic disease (Presta et al., U.S. Patent No. 5,965,709) and compounds that exhibit immunosuppressive activity and inhibits the release of histamine (Bycroft et al., U.S. Patent No. 5,969,158). St. Remy et al., U.S. Patent No. 4,740,371, discloses an immune complex of an allergen for treating allergies involving a combination of the specific allergen and the corresponding antibody to that allergen. The injection of the complex into a patient is said to reduce a patient's allergic reaction to that specific allergen. Others have suggested that certain human proteins can neutralize IgE by blocking it from interacting with the mast cells, but this has not been established clearly as a clinically effective therapy (Stanworth, et al., Allergy Treatment with a Peptide Vaccine, Lancet 1990; 336:1279-81). Patterson et al., U.S. Patent No. 5,314,690 disclosed a method and preparations for reducing IgE antibodies to allergens in allergic subjects wherein substance P (a neuropeptide) and an allergen or fragments of allergens or haptens acting as allergens are administered together to the allergic subjects through a non-oral route.

### Cholera Toxin and B Subunit as Adjutants

Cholera toxin (CT) and the closely related heat-labile toxin (LT) from *Escherichia coli* are known as exceptionally potent immunoadjuvants when co-administered with antigens by various mucosal routes (Elson et al., J. Immunol. 1984; 133:2892-2897; Holmgren et al., Vaccine 1973; 11:1179-1184; Lycke et al, Eur. J. Immunol. 1992; 22:2277-2281. Both CT and LT are recognized as "AB" toxins in that they are composed of two distinct structural and functional components: a single toxic-active A subunit component and a non-toxic cell-binding B subunit homopentamer component with strong binding affinity for GM1 ganglioside receptors (Holmgren et al., Nature 1981; 292:413-4117). Such receptors are known to be present on most mammalian cells, *e*.*g*., on skin and other epithelial cells, on all known antigen-presenting cells including dendritic cells (DC) and Langerhan's cells (LC), as well as on B and T lymphocytes.

Recently, Glenn et al., U.S. Patent No. 5,980,898 and WO99/43350 disclosed a system for transcutaneous immunization that induces an immune response (*e*.*g*., humoral and/or cellular effectors) in an animal or human. The system provides a simple application to intact skin of both rodents and humans of a formulation comprised of antigen and an adjuvant that was whole cholera toxin (CT) or LT to induce a specific antibody-mediated immune response against both the co-administered antigen and the toxin (Glenn et al., Nature 1998; 391:85; Glenn et al, Immun. 1999; 67:1100-1106; and Glenn et al., Nature Med. 1000; 6:1403-1406). This humoral immune response was generated without the causing the systemic toxicities attributable to the use of CT by other application routes Scharton-Kersten et al., disclosed transcutaneous immunization using CT or LT as adjuvants to generate a humoral response against diptheria toxin (Infection and Immunity 2000; 68:5306-5313). Collins et al., U.S. Patent No. 5,013,555 discloses an agent for desensitizing man and/or animals against an allergen comprising liposomes that comprise an allergen. Russell-Jones et al., U.S. Patent No. 6,103,243 discloses the mucosal presentation of an immunogen linked to a carrier molecule (heat labile toxin of enterotoxigenic *E*. *coli* (ETEC) or the binding subunit of heat labile toxin of ETEC), which is capable of specifically interacting with the mucosal epithelium of a vertebrate host and elicits a systemic, cellular and/or mucosal immune response in the vertebrate host. These findings are important in that they suggest that such adjuvanted transcutaneous immunization (TCI) could be a useful approach to efficacious needle-free vaccine delivery, which is a global priority because of the health hazards associated with the existing re-use of and unsafe disposal of injection needles.

Epicutaneous application of a prototype protein antigen (tetanus toxoid) together with CT or LT was shown to induce systemic antigen-specific proliferative T cell responses, characterized by a mixed Th1/Th2 phenotype, but with a clear Th2 bias (Hammond et al., Vaccine. 2001; 19:2701-2707). However, this bias for a Th2 type cytokine pattern could be a significant problem for the wide-spread use of TCI, since previous studies have described that various vaccine delivery approaches which favor Th2 responses all induced the production of high levels of reaginic immunoglobulins of IgE subclass and the appearance of atopic dermatitis (Spergel et al., J. Clin. Invest. 1988; 101:1614-1622; Wang et al., J. Immunol. 1986;156:4079-4082).

In contrast to their holotoxin counterparts (CT and LT), the less toxic B subunits (CTB and LTB), are highly efficient carrier molecules for the induction of mucosal antibody responses (Czerkinsky et al., Infect Immun. 1989; 57:1072-7, Holmgren et al., Walter de Gruyter, Berlin, New York 1996; Lipscombe et al., Mol. Microbiol. 1991; 5:1385-1392, McKenzie et al., J. Immunol. 1984; 133:1818-1824) as well as for the induction of mucosally induced systemic T cell (Sun et al., Proc. Natl. Acad. Sci. USA 1994; 91:10795-10799) and B cell oral tolerance (Rask et al., Clin. Exp. Allergy 2000; 30:1024-1032, Tamura et al., Vaccine 1997;15:225-229, Wiedermann et al., Int. Immunol. 1999;11:1717-1724) for various antigens physically linked to these proteins. However, CTB and LTB have been inefficient (oral immunization) or at best partly efficient (nasal immunization) as immunoadjuvants for unlinked co-administered antigens (Czerkinsky et al., Immunol. Rev. 170:197-222; Sun et al., Proc. Natl. Acad. Sci. USA 1994; 91:10795-10799). Further, the coupling of antigen to CTB (or LTB) for mucosal immunization, while promoting mucosal IgA immune response to the conjugated antigen, profoundly enhances the induction of systemic tolerance as opposed to abrogating systemic tolerance to the conjugated antigen as demonstrated using CT holotoxin (Elson et al, J. Immunol. 1984; 133:2892-2897).

The present inventors previously discovered that single oral administration of minute amounts of particulate or soluble antigen coupled to the B subunit of cholera toxin (CTB) can markedly suppress systemic immune responses in naive and in systemically immune animals.
This strategy of tolerance induction, based on oral administration of small amounts of antigens conjugated to a mucosa-binding molecule, is useful for preventing or abrogating unwanted immune responses (Sun JB, Holmgren J, and Czerkinsky C. et al., Proc Natl Acad Sci USA 1994; 91(23):10795-9).

The present inventors described results relating to the use of CT and its B subunit CTB as carrier molecules and adjuvants for promoting antigen presentation and increasing the immune response through their action on different antigen-presenting cells (APC) *ex vivo.* CTB was shown to be an efficient carrer molecule for ex-vivo antigen-pulsing of dendritic cells another APC, strongly promoting Th2 responses, as evidenced by vaccination of syngeneic mice with the pulsed APC, followed by boosting with the protein or peptide. Treatment of dendritic cells with CT as an adjuvant also markedly enhanced their immunostimulatory capacity, but predisposed also them for eliciting Th1 responses.

It was concluded that conjugation of an antigen to mucosa-binding molecules such as CTB and/or LTB can dramatically increase their mucosal immunogenicity and that this approach would prove useful in the preparation of mucosal vaccines (Rask C, Fredriksson M, Lindblad M, Czerkinsky C, and Holmgren J., APMIS, 108(3):178-86, 2000).

### SUMMARY OF THE INVENTION

The present description discloses a method for suppression an IgE-mediated allergic reaction in a mammal by transdermal administration of an allergen in conjuction with a non-ADP ribosylating toxin or toxin subunit in amounts effective to suppress the allergic reaction.

In one embodiment, the allergen is admixed with the non-ADP ribosylating toxin or toxin subunit.

In another embodiment, the allergen is chemically conjugated to the non-ADP ribosylating toxin or toxin subunit.

In yet a further embodiment, the allergen is genetically fused to the non-ADP ribosylating toxin or toxin subunit.

In another embodiment, the allergen and non-ADP-ribosylating toxin or subunit are comprised in a formulation suitable for topical or transcutaneous administration.

In yet another embodiment, the allergen and holotoxin comprising both A and B subunits are comprised in a formulation along with an aldehyde.

These and other aspects of the present invention will be apparent to those of ordinary skill in the art in light of the present specification, claims and drawings.

### BRIEF DESCRIPTION OF THE DRAWINGS

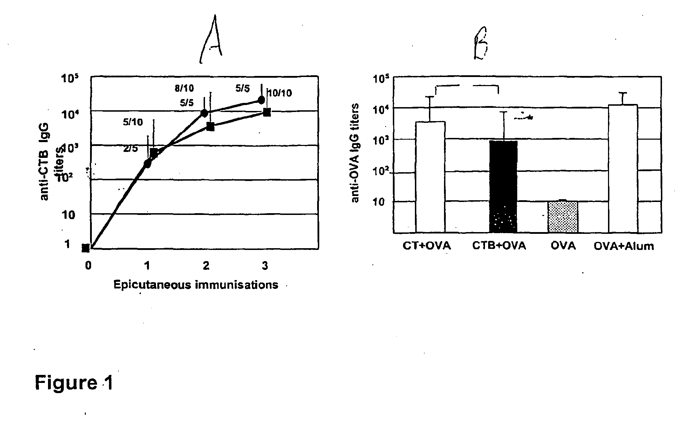

**Figure 1** **(A and B). Serum antibody responses after transcutaneous immunization with OVA together with CT or CTB.** Groups of 15 mice each received 3 topical applications of ovalbumin (300 µg) alone or co-administered with 50 µg of either CT (filled circles) or CTB (filled squares) at days 1, 14 and 21. An additional group of mice received an i.p. injection of 100 µg alum-adsorbed-OVA (as positive control) 3 weeks prior to analysis. Anti-CTB (A) and Anti-OVA (B) IgG were detected in serum 2 weeks after the first and second (A) and 3 weeks after the last epicutaneous immunization by ELISA assay. Data are expressed as IgG geometric mean titers plus one standard deviation and are represented in a logarithmic scale. * denotes a significant difference calculated by Wilcoxon's rank test between the comparison groups as p<0.05.

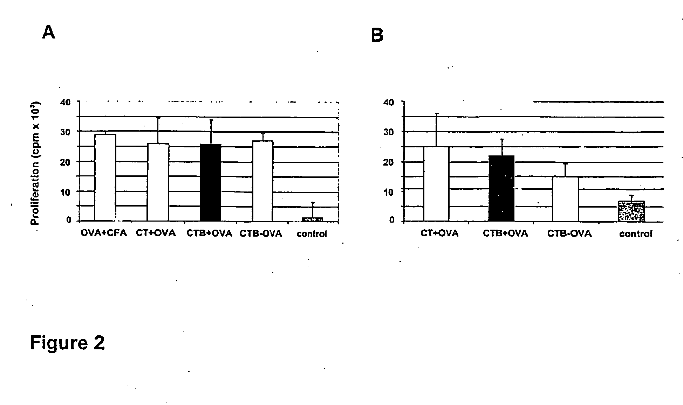

Figure 2(A and B). Regional (draining lymph nodes) and systemic (spleen) proliferative responses after epicutaneous immunization with OVA in presence of CT or CTB. Groups of mice received 3 topical applications of ovalbumin (300 µg) alone (control) or co-administered with 50 µg of either CT or CTB, or of CTB-OVA conjugate (300 µg) at days 1, 14 and 21. A group of mice received a subcutaneous (s.c.) injection of 100 µg OVA in complete Freund's adjuvant as positive control, 3 weeks prior to analysis. OVA-induced regional (A) and systemic (B) proliferative responses were determined 4 weeks after the last epicutaneous treatment by *in vitro* stimulation of draining lymph node cells and splenic cells with 2mg/ml OVA. Data are expressed as arithmetic mean (± SD) determined on groups of 15 individual mice.

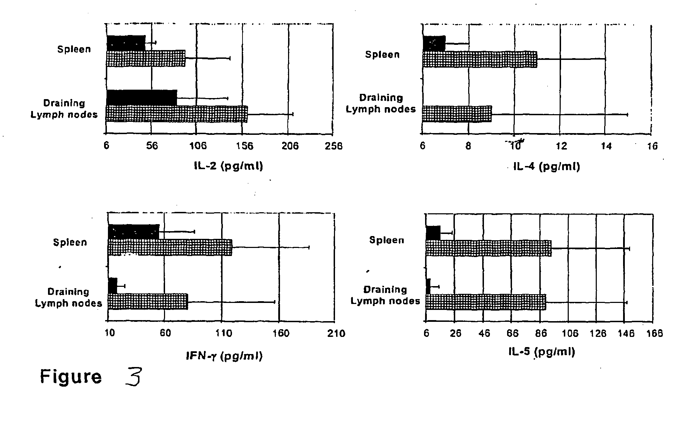

Figure 3. CT and CTB differentially affected Th1 and Th2 cytokine production by draining lymph node cells and spleen cells after epicutaneous immunization with ovalbumin in presence of CT or CTB. Cultures of draining lymph node or spleen cells from mice epicutaneously immunized with OVA co-administered either with CT (dashed bars) or CTB (white bars) as described in Figure 2 were performed in presence of OVA (2 mg/ml). Cytokine production in culture supernatants was measured after 24 h (IL-2) and 48 h (IFN-γ, amino IL-4 and IL-5) using a sandwich ELISA technique. Results are representative of three independent experiments.

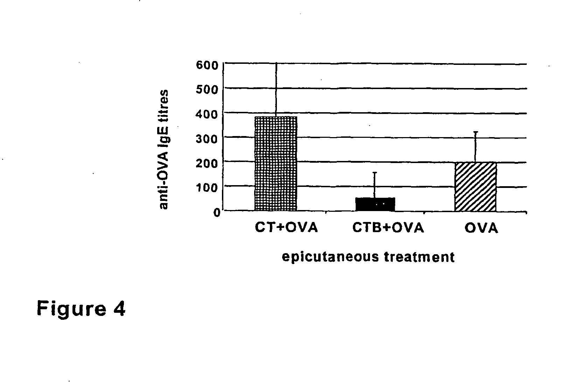

**Figure 4****. Prevention of systemic IgE responses by prior epicutaneous treatment with OVA in presence of CTB.** Groups of 8 mice in each received 6 topical applications of OVA (300 µg) either alone, or with 50 µg of CT or CTB every two days during two weeks and were challenged 3 weeks later by an i.p. injection of 100 µg of alum adsorbed OVA. Sera were taken 3 weeks after the sensitization and analyzed by passive cutaneous anaphylaxis titration (PCA) as described in material and methods. Results are representative of two independent experiments.

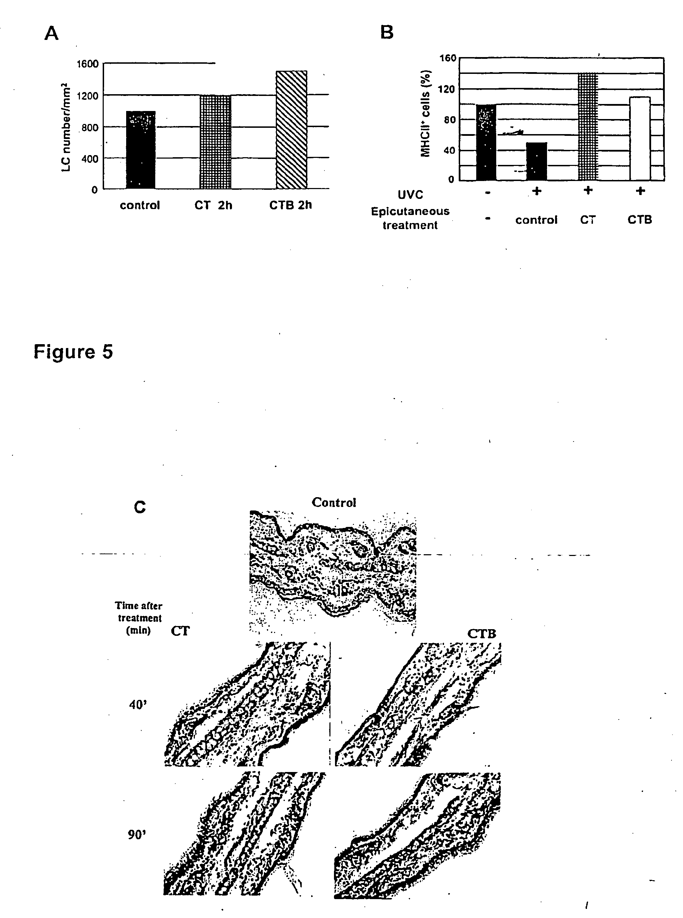

**Figure 5** **(A-C). Epicutaneous CT or CTB treatment increased LC numbers in epidermal sheets.** Groups of 3 mice in each were treated with 20 µg of CT or CTB dissolved in PBS containing 0.1% Tween 20 during one hour by skin application. (A) Ears were removed 90 minutes following epicutaneous treatment and epidermal sheets were prepared as described in material and methods. MHCII+ LCs were analyzed by immunohistochemical staining using the rat monoclonal antibody D9 conjugated to biotin specific for mouse IA-IE molecules, followed by enzymatic revelation with streptavidin-peroxidase and AEC as substrate and MHCII+ LCs were counted. Results are expressed as the mean number of cells/mm² derived from counting 10 fields per sample. (B) Ears were removed 90 minutes following epicutaneous treatment performed on mice which received an UV-C irradiation during 45 minutes 36 hours before, and epidermal cell suspensions were prepared as described in material and methods. Cell suspensions were obtained by mechanical extraction and were analyzed after double staining with FITC-conjugated anti-CD11c, PE-conjugated anti-MHCII. LC frequencies in CT and CTB-treated mice epidermal suspensions were compared to control mice and expressed in percentage, 100% corresponding to LC numbers in non-irradiated control mice. (C) Transverse paraffin-embedded sections of ears (10 mm) from control, CT- or CTB- treated mice were prepared at different times after epicutaneous treatment (40 min, 180 min) and immunohistochemical staining was performed as above to identify MHCII+ cells. Sections were counterstained with eosin-hematoxylin.

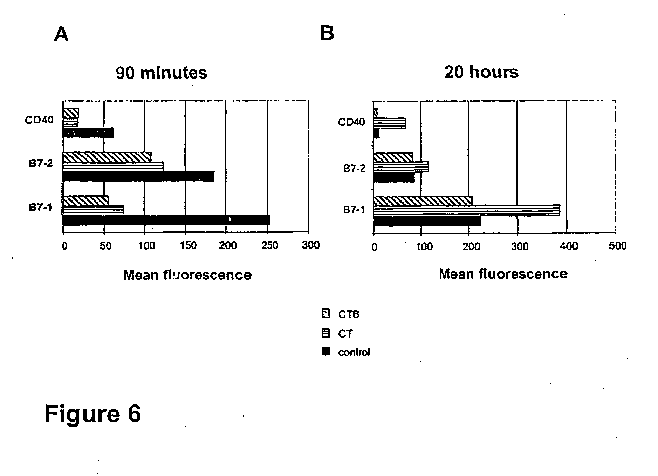

**Figure 6****. Epicutaneous treatment with CT but not with CTB induces up-regulation of co-stimulatory molecules B7-1 and B7-2 on epidermal dendritic cells.** Mice were treated either with CT (20 µg) (horizontal dashed bars), recombinant CTB (20 µg) (oblique dashed bars) or PBS (control) (black bars) and epidermal sheets were prepared either 90 minutes (A) or 24 hours (B) after epicutaneous treatment as described in material and methods. Cell suspensions were obtained by mechanical extraction and were analyzed after triple staining with FITC-conjugated anti-CD11c, PE-conjugated anti-NHCII, and biotin-conjugated antibodies against B7-1, B7-2 and CD40 molecules followed by streptavidin-tricolor. Results are expressed for MHCII+CD11c+ LCs as the mean fluorescence for each marker and are representative of three independent experiments.

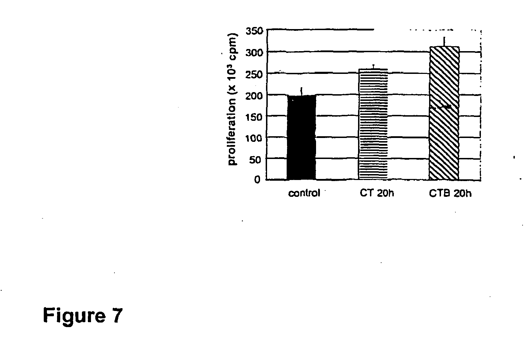

**Figure 7****. Epicutaneous CT and CTB treatments potentiated the antigen-specific proliferation of naive T cells by DLN DC.** DCs from auricular, inguinal and axillary draining lymph nodes were purified from animals treated by epicutaneous application of CT (horizontal dashed bars), CTB (oblique dashed bars) or PBS (black bars) 24 hours before preparation as described in material and methods. (A) Purified transgenic TCR-HA T cells from lymph nodes were cultured *in vitro* in flat-bottom 96-well plates at a concentration of 2x10⁵ T cells/well with 10⁴ DCs isolated from either CT-, CTB-treated or control mice in presence or not of HA peptide (aa 110-119) (5 mM) during 72 hours and pulsed with 1 mCi of [³H] thymidine during the last 18 hours of culture. Data are expressed as mean (+/-SD) of cpm from samples analyzed in triplicate. These results are representative of 3 individual experiments with similar results.

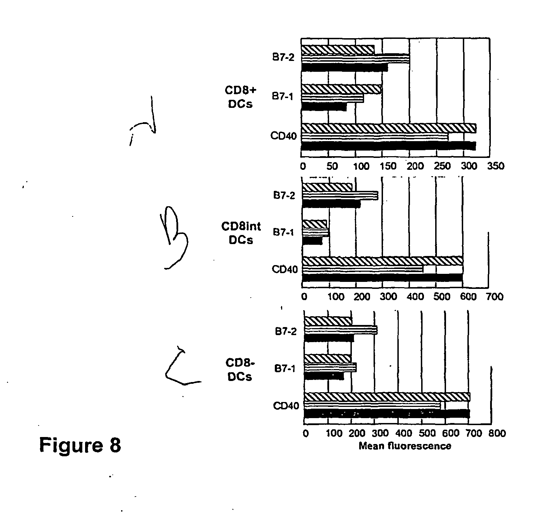

**Figure 8** **(A-C). Epicutaneous CT and CTB treatments differentially affected the expression of B7-1, B7-2 and CD40 co-stimulatory molecules on draining lymph dendritic cells.** DC from auricular, inguinal and axillary draining lymph nodes were purified from animals treated by epicutaneous application of CT (horizontal dashed bars), CTB (oblique dashed bars) or PBS (black bars) 24 hours before preparation as described in material and methods. DC were analyzed by cytometry after triple staining using FITC-conjugated anti-CD11c, PE-conjugated anti-CD8a and biotin-conjugated markers (anti-B7-1, anti-B7-2 and anti-CD40 monoclonal antibodies) followed by streptavidin tricolor. Expression of B7-1, B7-2 and CD40 by DC subsets was represented in bars showing the mean fluorescence of the different auricular DC subsets corresponding to gated CD8+ (A), CD8int (B) or CD8- (C) DCs from control CT- or CTB- skin treated mice. Most notable is a consistently increased expression of B7-2 by all DC subsets from CT treated mice.

### DETAILED DESCRIPTION OF THE INVENTION

It has now been unexpectedly shown that transcutaneous administration of CTB admixed or chemically or genetically conjugated to an allergen can suppress established, IgE-mediated allergic reactions This is particularly surprising in view of the fact that the transcutaneous administration of native CT holotoxin with the same antigen induces strong IgE responses to said antigen as would be expected from Glenn et al who showed that such an approach induces T cell responses with a clear bias toward Th2 responses whose archetype is IgE. The unexpected suppressive effect of CTB on IgE responses was associated with induction of a Th1-type T cell-mediated immune response and with a recruitment of dermal DC and an increase epidermal Langerhan's cells (LC) numbers.

Based on the markedly different and sometimes even opposing immuno-modulating effects of CT and CTB in many systems the effects of CT and CTB on the immune response to a co-administered prototype antigen, ovalbumin (OVA) in TCI were compared herein. It is demonstrated that while CTB can induce a substantial antibody response to itself when applied topically onto skin, it is relatively inefficient at inducing a systemic antibody response to the co-administered OVA antigen. By contrast, skin exposure to antigens conjugated or admixed with CTB evokes vigorous T cell proliferative responses to the heterologous antigen. Further, and different from CT which enhances the production of both Th1 and Th2 cytokines, and different from our findings described above in provisional application Serial No. 60/290,732, CTB promoted highly Th1 polarized responses. The adjuvant, activities of CT and CTB on cellular immune responses were associated with a rapid recruitment of immature DC in the epidermis and in the dermis. Taken together these results suggest that the adjuvant properties of CT for Th1 and Th2-driven responses in TCI involve structurally different moieties associated with its A and B subunits, respectively, and that CTB is useful as an immunoadjuvant in TCI for eliciting Th1-driven immune responses and, thus avoiding risks of IgE formation to co-administered antigens.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

As used herein, the phrase "allergen" encompasses fragments of allergens or haptens acting as allergens. These can include all the specific allergens that can cause an IgE mediated response in allergic subjects. This invention is therefore believed to be useful for the treatment of allergic diseases in humans, other primates, and mammalian subjects, such as dogs, cats, and horses.

The phrase "suppressing" means the complete or partial alleviation of the effects of an allergic response or type I hypersensitivity reaction.

As used herein, the phrase "hypersensitivity reactions" encompasses a state of altered reactivity in which the body reacts with an exaggerated immune response to a foreign substance such as an allergen.

"An amount effective to suppress" is that amount of allergen which modulates an allergic response. Such modulation of an immune response may provide a treatment such as, for example, desensitization, or immunosuppression.

As used herein, the phrase "transdermal" or "epicutaneous" or "topical" means entering through the dermis of the skin without perforation of the skin.

"Antigen presenting cells" (APC) are any mammalian cell which, either constitutively or after stimulation by adjacent cells or their products or by exogenous means, is capable of presenting antigen to cells of the immune system in a manner leading to an immune response. Examples of APC include but are not limited to different types and maturation stages of dendritic cells (DC), macrophages (MØ), B cells, Langerhan's cells, mast cells, and epithelial cells (the latter exemplified but not limited to epithelial cells derived from skin (keratinocytes), buccal epithelium, intestinal epithelium, or uro-genital tract epithelium).

"2angerhan's cells (LC)" are cells of dendritic appearance, strongly MHC Class II positive and weakly phagocytic, found in the basal layers of the epidermis where they serve as accessory cells, responsible for antigen processing and presentation. Once exposed to antigen they migrate to the lymph nodes. LC are derived from bone marrow and are immature dendritic cells. Part of the immune surveillance system, their location means that they are readily exposed to antigens that penetrate the epidermal barrier.

A "Th1" response is characterized by increased IFN-γ and IL-2 production as well a stronger induction of cytotoxic CD8+ T-cells.

A "Th2" response is characterized by production of certain classes and subclasses of antibodies such as IgG1, IgG2b or IgE, increased IL-4 and IL-5 production, and decreased IgG2a/IgG1 ratio.

"Cytotoxic T lymphocytes (CTLs)" are CD8+ "killer" T cells that bind epitopes that are part of or associated with class I histocompatibility molecules. They secrete molecules that destroy the cell to which they have bound. In addition to ridding the body of cells that have been infected by viruses or transformed by cancer, CTLs are responsible for the rejection of tissue and organ grafts.

"Co-stimulatory molecules" are involved in the interaction between receptor-ligand pairs expressed on the surface of antigen presenting cells and T cells. One exemplary receptor-ligand pair is the B7 co-stimulatory molecules on the surface of DCs and its counter-receptor CD28 or CTLA-4 on T cells (Freeman, et al. Science 1993; 262:909-911; Young, et al. J. Clin. Invest. 1992; 90: 229; Nabavi, et al. Nature 360:266). Other important co-stimulatory molecules are CD40, CD54, CD80, CD86.

The term "draining lymph node field" as used in the invention means an anatomic area over which the lymph collected is ciculating through a set of defined set of lymph nodes (*e*.*g*., cervical, axillary, inguinal, epitrochelear, popliteal, those of the abdomen and thorax).

The terms "major histocompatibility complex" or "MHC" refers to a complex of genes encoding cell-surface molecules that are required for antigen presentation to T cells and for rapid graft rejection. In humans, the MHC complex is also known as the HLA complex. The proteins encoded by the MHC complex are known as "MHC molecules" and are classified into class I and class II MHC molecules. Class I MHC molecules include membrane heterodimeric proteins made up of an alpha chain encoded in the MHC associated noncovalently with betamicroglobulin. Class I MHC molecules are expressed by nearly all nucleated cells and have been shown to function in antigen presentation to CD8+ T cells. Class I molecules include HLA-A, - B, and -C in humans. Class II MHC molecules also include membrane heterodimeric proteins consisting of non-covalently associated .alpha, and .beta. chains. Class II MHC are known to participate in antigen presentation to CD4+ T cells and, in humans, include HLA-DP, -DQ, and DR.

MHC class II molecules bind to peptides derived from proteins made outside of an antigen presenting cell. In contrast, MHC class I molecules bind to peptides derived from proteins made inside a cell. In order to present peptide in the context of a class II molecule, an APC phagocytoses an antigen into an intracellular vesicle, in which the antigen is cleaved, bound to an MHC class II molecule, and then returned to the surface of the antigen presenting cell.

The term "adjuvant" as used in the invention, is meant to describe a substance added to the formulation to assist in inducing an immune response to the antigen. "In conjunction with" is defined as a formulation between two or more reactants (such as an antigen or part thereof and an immunomodulating agent such as cholera toxin B subunit (CTB) or other toxin B subunits (*e*.*g*., *E. coli* LTB) parts thereof with or without added cytokines or other agents including immunomodulating nucleic acids or oligonucleotides), wherein these reactants are presented to an APC in conjunction with each other in any effective formulation including but not limited to a mixture, a chemical conjugate, a gene fusion protein or a nucleic acid preparation encoding for either or all of the reactants.

The term "transdermally" or "epicutaneous" or "topical" will be used interchangeably to describe the route of administration for the complexes of the present invention.

"Cholera toxin (CT)" is a bacterial exotoxin from the family of ADP-ribosylating exotoxins. Most ADP-ribsoylating exotoxins are functionally organized as A:B dimers with a binding B part or subunit and an A part or subunit containing the ADP-ribosyltransferase activity. Such toxins include diphtheria toxin, *Pseudomonas* exotoxin A, CT, *E. coli* heat-labile enterotoxin (LT), pertussis toxin (pertussigen), *C*. *botulinum* toxin C2, *C. botulinum* toxin C3, *C. limosum* exoenzyme, B. cereus exoenzyme, *Pseudomonas* exotoxin S, *Staphylococcus aureus* EDIN, and *B. splzaericus* toxin.

CT is the archetype example of an ADP-ribsoylating exotoxin that is organized with A and B subunits. The B subunit is the binding subunit and consists of a B-subunit homopentamer which is non-covalently bound to the single-subunit A subunit. The B-subunit pentamer is arranged in a symmetrical doughnut-shaped structure that binds to GM1 -ganglioside on the target cell. The A subunit serves to ADP ribosylate the alpha subunit of a subset of the hetero trimeric GTP proteins (G proteins) including the Gs protein which results in the elevated intracellular levels of cyclic AMP. This stimulates release of ions and fluid from intestinal cells in the case of cholera.

Cholera toxin and its B subunit (CTB) have adjuvant properties when used as either an intramuscular or oral immunogen (Elson and Dertzbaugh, J Immunol. 1995;154(3):1032-40.; Trach et al., Lancet. 1997; 349(9047):231-5.). Another antigen, heat-labile enterotoxin from *E*. *coli* (LT) is 80% homologous at the amino acid level with CT and possesses similar binding properties; it also appears to bind the GM1 -ganglioside receptor in the gut and has similar ADP-ribosylating exotoxin activities. Another ADP-ribosylating exotoxin, Pseudomonas exotoxin A (ETA), binds to the alpha-2 -macroglobulin receptor-low density lipoprotein receptor-related protein (Kounnas et al., J Biol Chem. 1993; 268(19):14176-81.). ADP-ribsoylating exotoxins are reviewed by Krueger and Barbieri, Clin Microbiol Rev. 1995; 8(1): 34-47. However, an important difference between CT and CTB described herein, is that the B subunit, and other non-ADP-ribsoylating exotoxins such as the B subunit of *E*. *coli* LT when administered transdermally with an allergen, suppresses the allergic reaction.

CTB and derivatives are defined as any protein which either binds to GM1 ganglioside and/or reacts with polyclonal antiserum to CTB as detected by an ELISA or GM1-ELISA test, including but not limited to B subunit of heat-labile enterotoxin from *Escherichia coli* B (LTB) and to any or all mutated, extended, truncated or otherwise modified forms of B subunits or any other protein that would react with GM1 or with said types of anti-sera as well as any nucleic acid preparation that would encode for a protein that would meet these criteria. CTB and derivatives do not have ADP-ribosylating activity.

### Molecular Biology Definitions

The term "about" or "approximately" means within an acceptable error range for the particular value as determined by one of ordinary skill in the art, which will depend in part on how the value is measured or determined, *i*.*e*., the limitations of the measurement system. For example, "about" can mean within 1 or more than 1 standard deviations, per the practice in the art. Alternatively, "about" can mean a range of up to 20%, preferably up to 10%, more preferably up to 5%, and more preferably still up to 1 % of a given value. Alternatively, particularly with respect to biological systems or processes, the term can mean within an order of magnitude, preferably within 5-fold, and more preferably within 2-fold, of a value.
The terms "mutant" and "mutation" mean any detectable change in genetic material, *e*.*g*., DNA, or any process, mechanism or result of such a change. This includes gene mutations, in which the structure (*e*.*g*., DNA sequence) of a gene is altered, any gene or DNA arising from any mutation process, and any expression product (*e*.*g*., RNA, protein or enzyme) expressed by a modified gene or DNA sequence. The term "variant" may also be used to indicate a modified or altered gene, DNA sequence, RNA, enzyme, cell, *etc*.; *i*.*e*., any kind of mutant.

An "analog" is a substance that can mimic the biological effects of the non-ADP ribosylating toxin subunit when administered transdermally, including immunouppressive activity, that is characteristic of a non-ADP ribosylating toxin subunit.

The term "purified" as used herein refers to material that has been isolated under conditions that reduce or eliminate the presence of unrelated materials, i.e., contaminants, including native materials from which the material is obtained. For example, a purified protein is preferably substantially free of other proteins or nucleic acids with which it is associated in a cell; a purified nucleic acid molecule is preferably substantially free of proteins or other unrelated nucleic acid molecules with which it can be found within a cell. As used herein, the term "substantially free" is used operationally, in the context of analytical testing of the material. Preferably, purified material substantially free of contaminants is at least 50% pure; more preferably, at least 90% pure, and more preferably still at least 99% pure. Purity can be evaluated by chromatography, gel electrophoresis, immunoassay, composition analysis, biological assay, and other methods known in the art.

Methods for purification are well known in the art. For example, nucleic acids can be purified by precipitation, chromatography (including preparative solid phase chromatography, oligonucleotide hybridization, and triple helix chromatography), ultracentrifugation, and other means. Polypeptides and proteins can be purified by various methods including, without limitation, preparative disc-gel electrophoresis, isoelectric focusing, HPLC, reversed-phase HPLC, gel filtration, ion exchange and partition chromatography, precipitation and salting-out chromatography, extraction, and countercurrent distribution. For some purposes, it is preferable to produce the polypeptide in a recombinant system in which the protein contains an additional sequence tag that facilitates purification, such as, but not limited to, a polyhistidine sequence, or a sequence that specifically binds to an antibody, such as FLAG and GST. The polypeptide can then be purified from a crude lysate of the host cell by chromatography on an appropriate solid-phase matrix. Alternatively, antibodies produced against the protein or against peptides derived therefrom can be used as purification reagents. Cells can be purified by various techniques, including centrifugation, matrix separation (*e*.*g*., nylon wool separation), panning and other immunoselection techniques, depletion (*e*.*g*., complement depletion of contaminating cells), and cell sorting (*e*.*g*., fluorescence activated cell sorting [FACS]). Other purification methods are possible. A purified material may contain less than about 50%, preferably less than about 75%, and most preferably less than about 90%, of the cellular components with which it was originally associated. The "substantially pure" indicates the highest degree of purity which can be achieved using conventional purification techniques known in the art.

A "coding sequence" or a sequence "encoding" an expression product, such as a RNA, polypeptide, protein or enzyme, is a nucleotide sequence that, when expressed, results in the production of that RNA, polypeptide, protein or enzyme; *i*.*e*., the nucleotide sequence "encodes" that RNA or it encodes the amino acid sequence for that polypeptide, protein or enzyme.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently found, for example, by mapping with nuclease S1), as well as protein binding domains (consensus sequences) responsible for the binding of RNA polymerase.

A coding sequence is "under the control of" or is "operatively associated with" transcriptional and translational control sequences in a cell when RNA polymerase transcribes the coding sequence into RNA, which is then trans-RNA spliced (if it contains introns) and, if the sequence encodes a protein, is translated into that protein.

The term "express" and "expression" means allowing or causing the information in a gene or DNA sequence to become manifest, for example producing RNA (such as rRNA or mRNA) or a protein by activating the cellular functions involved in transcription and translation of a corresponding gene or DNA sequence. A DNA sequence is expressed by a cell to form an "expression product" such as an RNA (e.g., a RNA or a rRNA) or a protein. The expression product itself, e.g., the resulting RNA or protein, may also said to be "expressed" by the cell.

The term "transfection" means the introduction of a foreign nucleic acid into a cell. The term "transformation" means the introduction of a "foreign" (i.e., extrinsic or extracellular) gene, DNA or RNA sequence into a host cell so that the host cell will express the introduced gene or sequence to produce a desired substance, in this invention typically an RNA coded by the introduced gene or sequence, but also a protein or an enzyme coded by the introduced gene or sequence. The introduced gene or sequence may also be called a "cloned" or "foreign" gene or sequence, may include regulatory or control sequences (e.g., start; stop, promoter, signal, secretion or other sequences used by a cell's genetic machinery). The gene or sequence may include nonfunctional sequences or sequences with no known function. A host cell that receives and expresses introduced DNA or RNA has been "transformed" and is a "transformant" or a "clone". The DNA or RNA introduced to a host cell can come from any source, including cells of the same genus or species as the host cell or cells of a different genus or species.

The terms "vector", "cloning vector" and "expression vector" mean the vehicle by which a DNA or RNA sequence (e.g., a foreign gene) can be introduced into a host cell so as to transform the host and promote expression (e.g., transcription and translation) of the introduced sequence. Vectors may include plasmids, phages, viruses, etc. and are discussed in greater detail below.

Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct." A common type of vector is a "plasmid", which generally is a self-contained molecule of double-stranded DNA, usually of bacterial origin, that can readily accept additional (foreign) DNA and which can readily introduced into a suitable host cell. A large number of vectors, including plasmid and fungal vectors, have been described for replication and/or expression in a variety of eukaryotic and prokaryotic hosts. The term "host cell" means any cell of any organism that is selected, modified, transformed, grown or used or manipulated in any way for the production of a substance by that cell. For example, a host cell may be one that is manipulated to express a particular gene, a DNA or RNA sequence, a protein or an enzyme. Host cells can further be used for screening or other assays that are described *infra*. Host cells may be cultured *in vitro* or one or more cells in a non-human animal (*e*.*g*., a transgenic animal or a transiently transfected animal).

A wide variety of host/expression vector combinations (*i*.*e*., expression systems) may be employed in expressing the DNA sequences of this invention. Useful expression vectors, for example, may consist of segments of chromosomal, non-chromosomal and synthetic DNA sequences. Suitable vectors include derivatives of SV40 and known bacterial plasmids, *e*.*g*., E. coli plasmids col El, pCR1, pBR322, pMa1-C2, pET, pGEX (Smith et al., Gene 67:31-40, 1988), pMB9 and their derivatives, plasmids such as RP4; phage DNAS, *e*.*g*., the numerous derivatives of phage 1, *e*.*g*., NM989, and other phage DNA, e.g., M13 and filamentous single stranded phage DNA; yeast plasmids such as the 2m plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like. In addition, various tumor cells lines can be used in expression systems of the invention.

Yeast expression systems can also be used according to the invention to express any protein of interest. For example, the non-fusion pYES2 vector (XbaI, SphI, ShoI, NotI, GstXI, EcoRI, BstXI, BamH1, SacI, Kpn1, and HindDI cloning sit; Invitrogen) or the fusion YESHisA, B, C (XbaI, SphI, ShoI, NotI, BstXI, EcoRI, BamH1, SacI, KpnI, and HindIII cloning site, N-terminal peptide purified with ProBond resin and cleaved with enterokinase; Invitrogen), to mention just two, can be employed according to the invention.

Expression of the protein or peptide may be controlled by any promoter/enhancer element known in the art, but these regulatory elements must be functional in the host selected for expression. Promoters which may be used to control gene expression include, but are not limited to, cytomegalovirus (CMV) promoter (U.S. Patent Nos. 5,385,839 and No. 5,168,062), the SV40 early promoter region (Benoist and Chambon, 1981, Nature 290:304-310), the promoter contained in the 3' long terminal repeat of Rous sarcoma virus (Yamamoto, et al., Cell 22:787-797, 1980), the herpes thymidine kinase promoter (Wagner et al., Proc. Natl. Acad. Sci. U.S.A. 78:1441-1445, 1981), the regulatory sequences of the metallothionein gene (Brinster et al., Nature 296:39-42; 1982); prokaryotic expression vectors such as the β-lactamase promoter (Villa-Komaroff, et al., Proc. Natl. Acad. Sci. U.S.A. 75:3727-3731, 1978), or the tac promoter (DeBoer, et al., Proc. Natl. Acad. Sci. U.S.A. 1983; 80:21-25); see also "Useful proteins from recombinant bacteria" in Scientific American, 242:74-94, 1980; promoter elements from yeast or other fungi such as the Gal 4 promoter, the ADC (alcohol dehydrogenase) promoter, PGK (phosphoglycerol kinase) promoter, alkaline phosphatase promoter; and transcriptional control regions that exhibit hematopoietic tissue specificity, in particular: beta-globin gene control region which is active in myeloid cells (Mogram et al., Nature 315:338-340, 1985; Kollias et al., Cell 46:89-94, 1986), hematopoietic stem cell differentiation factor promoters, erythropoietin receptor promoter (Maouche et al., Blood, 15:2557, 1991), etc.

Preferred vectors, particularly for cellular assays *in vitro* and *in vivo*, are viral vectors, such as lentiviruses, retroviruses, herpes viruses, adenoviruses, adeno-associated viruses, vaccinia virus, baculovirus, and other recombinant viruses with desirable cellular tropism. Thus, a gene encoding a functional or mutant protein or polypeptide domain fragment thereof can be introduced *in vivo*, *ex vivo*, or *in vitro* using a viral vector or through direct introduction of DNA. Expression in targeted tissues can be effected by targeting the transgenic vector to specific cells, such as with a viral vector or a receptor ligand, or by using a tissue-specific promoter, or both. Targeted gene delivery is described in International Patent Publication WO 95/28494, published October 1995.

Viral vectors commonly used for *in vivo* or *ex vivo* targeting and therapy procedures are DNA-based vectors and retroviral vectors. Methods for constructing and using viral vectors are known in the art (see, *e*.*g*., Miller and Rosman, BioTechniques, 7:980-990, 1992). Preferably, the viral vectors are replication defective, that is, they are unable to replicate autonomously in the target cell. Preferably, the replication defective virus is a minimal virus, *i*.*e*., it retains only the sequences of its genome which are necessary for encapsidating the genome to produce viral particles.

The term "expression system" means a host cell and compatible vector under suitable conditions, *e*.*g*., for the expression of a protein coded for by foreign DNA carried by the vector and introduced to the host cell. Common expression systems include E. coli host cells and plasmid vectors, insect host cells such as Sf9, Hi5 or S2 cells and Baculovirus vectors, Drosophila cells (Schneider cells) and expression systems, fish cells and expression systems (including, for example, RTH-149 cells from rainbow trout, which are available from the American Type Culture Collection and have been assigned the accession no. CRL-1710) and mammalian host cells and vectors.

The term "heterologous" refers to a combination of elements not naturally occurring. For example, the present invention includes chimeric RNA molecules that comprise an rRNA sequence and a heterologous RNA sequence which is not part of the rRNA sequence. In this context, the heterologous RNA sequence refers to an RNA sequence that is not naturally located within the ribosomal RNA sequence. Alternatively, the heterologous RNA sequence may be naturally located within the ribosomal RNA sequence, but is found at a location in the rRNA sequence where it does not naturally occur. As another example, heterologous DNA refers to DNA that is not naturally located in the cell, or in a chromosomal site of the cell. Preferably, heterologous DNA includes a gene foreign to the cell. A heterologous expression regulatory element is a regulatory element operatively associated with a different gene that the one it is operatively associated with in nature.

In one embodiment, the peptides or proteins are chemically coupled or genetically fused to CTB. Methods for chemically coupling peptides and proteins to CTB are set forth in the "Methods" section below. Any method of chemical coupling may be used. Preferably the coupling uses commercially available functional adaptors. These are all commercially available from numerous sources such as Sigma Chemical Co. (St. Louis, MO) Pharmacia, and Merck (White House Station, NJ). Peptide-protein carrier polymers may be formed using conventional cross-linking agents such as carbodiimides.

### Skin as an Immune Organ

The skin epithelium, the epidermis, is a stratified squamous keratinized epithelium formed by multilayers of keratinocytes (KC), covered by a corneal layer preventing absorption of macromolecules and pathogens. The skin contains professional antigen-presenting dendritic cells, including Langerhans cells (LC) in the epidermis and dermal dendritic cells (DC) in the dermis. LC, morphologically characterized by their long and slender cytoplasmic processes, from a network of cells protruding their dendrites between KC. Following antigen entry through the skin, LC are rapidly recruited from dermal DC precursors to the epidermis, where they capture and internalize the antigen by endocytosis and start processing native antigen into peptides which are retained in MHC class II (MHCII) vesicles of the endocytic pathway. Antigen capture by LC induces morphological changes and mobilization of the LC, which emigrate through afferent lymphatics to the T cell area of draining lymph nodes (DLN), where they can be identified as interdigitating DC, forming close contacts with lymph node T cells. At this stage they have acquired expression of co-stimulatory molecules of the B7 family, upregulated surface expression of MHC class I and class II molecules bound to peptides, and the ability to secrete high levels of pro-inflammatory cytokines such as IL-12, IL-1. Thus, interdigitating DC are highly specialized in the activation of naive T cells into antigen-specific T cells.

During recent years, it has become increasingly clear that manipulation of the immune system for therapeutic (*i*.*e*. anti-tumoral) or vaccination (anti-infectious) purposes, requires immunization routes allowing efficient antigen uptake by DC. The feasibility of antigen delivery through the skin for the induction of protective immunity against infections has emerged from the field of vaccinology and more recently illustrated by the delivery of sub-unit vaccines. The concept of sub-unit vaccines is based on the idea that harmful complications and potential reversion to virulence, which may occur after vaccination with live attenuated virus or bacteria, thereby limiting their use in immunocompromised individuals, may be overcome by vaccination with antigenic components of the pathogens in the form of recombinant proteins, synthetic peptides, or DNA encoding for the antigen of interest.

### Cell-Mediated Immune Response

The first step in antigen-specific T cell activation is controlled by antigen-presenting cells (APC) that adsorb, process and present antigens in a complex with MHC class II on the cells surface together with co-stimulatory signals. The responding T cells then are of the CD4+ subset and may develop into either Th1 or Th2 or other helper or regulatory cells. APCcs can also present antigens in a complex with either MHC class I or, more recently described, CD1 molecules on the cell surface. Antigens presented in the context of MHC class I are usually produced intracellularly in the APC, *e*.*g*., as a result of an infection with an intracellular bacterial, viral, or parasitic antigen (including recombinant such organisms engineered to express heterologous antigens intracellularly), and are recognized by cognate CD8+ T cells that may develop into cytotoxic lymphocytes (CTL). However, some forms of exogenous antigens, including but not limited to particulate antigen formulations can also be translocated into the APC MHC class I pathway and stimulate CD8+ T cells, including CTLs. The CD1 family of proteins are prominently expressed on most APC and these proteins have recently been found to provide yet another antigen presenting system in addition to that of MHC class I and MHC class II, by presenting predominantly various lipid and glycolipid antigens to CD 1 reactive/restricted T cells, including CD4+, CD8+, and CD4/CD8 double negative T cells and cells.

APCs utilize multiple mechanisms for antigen uptake, which vary according to cell type. Dendritic cells (DC) and macrophages (MØ) have a broad range of binding specificities through Fc-receptors and C-type multilectin receptors and can also absorb antigens by macropinocytosis and phagocytosis (Banchereau et al., Nature 1998; 392:245-252). Besides the nature of the antigen, the nature of the APC involved and the cytokines present and/or induced are important determinants for the outcome of the subsequent T cell response. Thus, the level of T cell activation depends on the densities of specific peptide-loaded MHC class II and of co-stimulatory molecules such as CD40, CD80 and CD86 present on the APC surface (McAdam et al., Immunol. Rev. 1998; 165:231-247, van Gool et al. Immunol. Rev. 1996; 153:47-83) as well as on the levels of cytokines produced such as IL-1, IL-12, and IL-18 (Kohno et al. J. Immunol. 1997; 158:1541-1550, O'Garra et al. Immunity. 1998; 8:275-283, Weaver et al., Immunol. Today 1990; 11:49-55).

### Transdermal Administration and Delivery

Previously, transdermal administration delivered agents to specialized cells (*e*.*g*., antigen-presenting cells (Langerhans cells) and lymphocytes) to induce an immune response (Bos, Clin Exp Immunol. 1997;107 Suppl 1:3-5). However, the present invention is based on, in part, the unexpected discovery that as well as inducing specific immune responses, the transdermal administration of allergens can result in the suppression of an ongoing allergic response.

One aspect of the present invention provides a novel means for modulating a hypersensitivity reaction through intact skin without the need for perforating the skin. The transdermal delivery system provides a method whereby allergens and carriers can be delivered to the immune system, to especially specialized antigen presentation cells underlying the skin such as, for example, Langerhans cells.

Without being bound to any particular theory but only to provide an explanation for observations made, it is presumed that the transdermal delivery system carries allergens to cells of the immune system where an allergic response is modulated. The antigen may pass through the normal protective outer layers of the skin (*i*.*e*., stratum corneum) and modulate the allergic response directly, or through an antigen presenting cell (*e*.*g*., macrophage, tissue macrophage, Langerhans cell, dendritic cell, dermal dendritic cell, or B lymphocyte) that presents processed antigen to a T lymphocyte. Optionally, the antigen may pass through the stratum corneum via a hair follicle or a skin organelle (*e*.*g*., sweat gland, oil gland).

Transdermal delivery systems can target immune cells more efficiently than the use of drugs because several locations can be targeted in large surface areas of skin. An effective amount of an allergen to suppress a hypersensitivity reaction may be delivered transdermally either at a single location, or over an area of intact skin covering multiple draining lymph node fields (*e*.*g*., cervical, axillary, inguinal, epitrochelear, popliteal, those of the abdomen and thorax). Such locations close to numerous different lymphatic nodes at locations all over the body will provide a more widespread access to the immune system than when a small amount of allergen is injected at a single location by intradermal subcutaneous or intramuscular injection.

The present invention provides a treatment which is both simple and east for the modulation of allergic responses. Transdermal delivery does not involve penetration of the skin, therefore the requirements of trained personnel, sterile techniques, and sterile equipment are reduced significantly, thereby reducing cost in healthcare.

Transdermal delivery may be achieved using a solution of allergen admixed or conjugated to CTB impregnated in gauze under a patch, or by using other patch technologies. Transdermal patches are well known in the art. See *e*.*g*., U.S. Patent Nos. 4,994,267; 5,605,701; 5,948,433 and 6,267,983. Processes for preparing a pharmaceutical formulation for transdermal delivery are well known in the art, whereby the antigen and adjuvant are combined with a pharmaceutically acceptable carrier vehicle. Suitable vehicles and their preparation are described, for example, in Remington's Pharmaceutical Sciences by E. W. Martin. Such formulations will contain an effective amount of the antigen and adjuvant together with a suitable amount of vehicle in order to prepare pharmaceutically acceptable compositions suitable for administration to a human or animal. The formulation may be applied in the form of a cream, emulsion, gel, lotion, ointment, paste, solution, suspension, spray or other forms known in the art. In particular, formulations that enhance skin hydration, penetration, or both are preferred. There may also be incorporated other pharmaceutically acceptable additives or excipients including, for example, diluents, binders, stabilizers, preservatives, penetration enhancers and colorings. Furthermore the transdermal delivery system could be given by untrained personnel, and is also amenable to self-application. Additionally, this simple delivery system would improve access to treatment of pediatric patients and the elderly, and populations in Third World countries.

In one embodiment of the present invention, liposomes may be used to delivery allergen and CTB. Liposomes are closed vesicles surrounding an internal aqueous space. The internal compartment is separated from the external medium by a lipid bilayer composed of discrete lipid molecules. In the present invention, allergens may be delivered through intact skin to specialized cells of the immune system. Transdermal delivery of allergens may be achieved by using liposomes; however, as shown in the examples, liposomes are not required to elicit an antigen-specific immune response.

The transdermal delivery system may be applied directly to the skin and allowed to air dry; rubbed into the skin or scalp; held in place with a dressing, patch, or absorbent material or sprayed onto the skin to maximize contact with the skin. The formulation may be applied in an absorbent dressing or gauze. The formulation may be covered with an occlusive dressing such as, for example, AQUAPHOR (an emulsion of petrolatum, mineral oil, mineral wax, wool wax, panthenol, bisabol, and glycerin from Beiersdorf, Inc.), plastic film, COMFEEL (Coloplast) or vaseline; or a non-occlusive dressing such as, for example, DUODERM (3M) or OPSITE (Smith & Napheu). An occlusive dressing completely excludes the passage of water.

The formulation may be applied to single or multiple sites, to single or multiple limbs, or to large surface areas of the skin by complete immersion. The formulation may be applied directly to the skin.

An advantage to using transdermal administration, in addition to eliminating the need for injection, is that lower doses of allergens can be used to achieve improved treatment of allergic responses compared to oral routes for therapy. Transdermal administration also makes it simpler to administer allergens to the patient, avoids gastrointestinal tract difficulties during absorption that may be caused by oral administration, and avoids first pass *i*.*e*. the initial passage of a drug substance through the systemic and portal circulation. Transdermal administration also has the capacity for multi-day therapy with a single application thereby improving patient compliance. Compared to subcutaneous immunotherapy, transdermal administration is a non- invasive method and does not require special facilities and trained personnel.

### Allergens

Foreign compounds that induce symptoms of immediate and/or delayed hypersensitivity are herein referred to as allergens. The term "allergen" primarily refers to foreign compounds capable of causing an allergic response. Factors that influence an animal's susceptibility to an allergen can include a genetic component and/or environmental exposure to an allergen. Animals can be de-sensitized to an allergen by repeated injections of the allergen to which an animal is hypersensitive.

Allergens encompassed by the present invention are: environmental aeroallergens, weed pollen allergens, grass pollen allergens, tree pollen allergens, house dust mite allergens, storage mite allergens, mold spore allergens, animal allergens, food allergens (*e*.*g*., egg, peanut, wheat, milk, seafood, fruit), insect allergens, venoms (*Hymenoptera*, yellow jacket, honey bee, wasp, hornet, fire ant) and other environmental insect allergens from cockroaches, fleas, mosquitoes, etc., bacteria such as streptococcal antigens, parasites such as *Ascaris* antigen, viral antigens, drug allergens, antibiotics, whole proteins such as hormones (insulin), enzymes (Streptokinase), and their metabolites capable of acting as incomplete antigens or haptens, industrial chemicals and metabolites capable of acting as haptens and stimulating the immune system, occupational allergens such as flour in Baker's asthma, castor bean, coffee bean, latex, cotton, wool, mineral oils, industrial dyes, metals including copper and cobalt (see Lachowsky et al., Curr Allergy Asthma Rep. 2001;1(6):587-93; Stenton, Occup Med. 2000;15(2):431-44; and Quirce et al., Allergy. 1998;53(7):633-41). See the following review articles for a detailed description of such allergens: Arruda LK, et al., Curr Allergy Asthma Rep. 2001; 1(5):466-73; Phipatanakul W., Curr Allergy Asthma Rep. 2001; 1(5):461-5. Review; Dziadzio L, Curr Allergy Asthma Rep. 2001; 1(5):455-60; Wurtzen P A., APMIS. 2001; 109(9):561-71;Fountain DW. et al, Biologist (London). 2002; 49(1):5-9; Zucker-Pinchoff B, Mt Sinai J Med. 2002;69(1-2):88-95; Spergel JM et al., Curr Opin Pediatr. 2001;13(6):517-22; Esch RE et al., Clin Rev Allergy Immunol. 2001 ;21(2-3):261-92.

A comprehensive disclosure of allergens and references or GenBank accession numbers where their nucleic acid and amino acid sequences can be found is in Appendix A of PCT WO 01/66136 to Panacea Pharmaceuticals, LLC, published on September 13, 2001.

Allergens of the invention may be expressed by recombinant means; chemical synthesis of an oligopeptide, either free or conjugated to carrier proteins, may be used to obtain antigen of the invention. Methods of generating recombinant polypetides are described in *e*.*g*., Sambrook, Fritsch & Maniatis, Molecular Cloning: A Laboratory Manual, Second Edition (1989) Cold Spring Harbor Laboratory Press, Cold Spring Harbor, New York; DNA Cloning: A Practical Approach, Volumes I and II (D.N. Glover ed. 1985); Oligonucleotide Synthesis (M.J. Gait ed. 1984); Nucleic Acid Hybridization [B.D. Hams & SJ.ÊHiggins eds. (1985)]; Transcription And Translation [B.D. Hames & S.J. Higgins, eds. (1984)]; Animal Cell Culture [R.I. Freshney, ed. (1986)]; Immobilized Cells And Enzymes [IRL Press, (1986)]; B.ÊPerbal, A Practical Guide To Molecular Cloning (1984); F.M. Ausubel et al. (eds.), Current Protocols in Molecular Biology, John Wiley & Sons, Inc. (1994).

A non-limiting example of a genetic fusion of an immunogenetic peptide and allergen, OVA, with CTA is described below in the Methods section.

Allergens obtained through recombinant means or peptide synthesis, as well as antigens of the invention obtained from natural sources or extracts, may be purified by means of the antigen's physical and chemical characteristics, preferably by fractionation or chromatography (Gregroire et al., Clin Rev Allergy Immunol. 2001;21(2-3):215-27). Alternatively, non-purified, crude allergens may also be used.

In one embodiment of the present invention, combinations of two or more allergens linked or fused to a toxin subunit that is devoid of ADP-ribosylating activity may be used. Alternatively, combinations of B subunit-linked and fused allergens are also contemplated. A formulation containing multiple allergens may be used to induce an immune response to more than one allergen at the same time.

The allergen may be solubilized in a buffer. Suitable buffers include, but are not limited to, phosphate buffered saline Ca 2+/Mg 2+ free (PBS), normal saline (150 mM NaCl in water), and Tris buffer. Glycerol may be a suitable non-aqueous buffer for use in the present invention.

### Cholera Toxin Subunits

Oral tolerance is a long recognized method for inducing systemic immunological tolerance. However, large doses of antigen and frequent administrations are often required. According to the present invention, it is demonstrated that when an antigen is linked to the nontoxic mucosa-binding CTB, the required amount of antigen can be dramatically reduced. The present inventors further discovered that oral prophylactic or therapeutic administration of a model allergen coupled to CTB modulates allergen-specific IgE responses in high IgE responder Balb/c mice. Therefore oral treatment with the CTB-OVA conjugate effectively suppressed antigen-specific Th1-mediated delayed-type hypersensitivity and treatment with a CTB-conjugated model allergen can affect a broad range of T cell-driven immune responses, even in antigen-experienced animals. (Rask C, Holmgren J, Fredriksson M, Lindblad M, Nordstrom I, Sun JB, and Czerkinsky C., Clin Exp Allergy, Jul;30(7):1024-32, 2000).

The present invention encompasses mucosa-binding molecules or mucosa-binding subunits or domains of toxin (such as cholera or *E. coli* LT), the most preferred mucosa-binding subunits is pure cholera toxin B subunit (CTB). "Pure" as used in this context means that the cholera toxin B subunit is essentially free of detectable contamination by active cholera toxin, which comprises a cholera toxin A subunit (CTA) in combination with CTB. An "active" cholera toxin molecule as used herein denotes one that exhibits ADP-ribosylating activity. Functional purity of CTB subunit for use in the present invention can be achieved by expressing the gene encoding cholera toxin B subunit in a bacterial cell (such as, for example, *V*. *cholerae*) in the absence of a gene encoding CTA. Methods for large-scale expression of pure CTB are disclosed in, for example, U.S. Patent No. 5,268,276. As shown in Holmgren et al. U.S. Patent No. 5,681,571, the present inventors have found that the presence of even small amounts of contaminating cholera toxin A subunit can abrogate the tolerance induced by the compositions of the present invention.

In another embodiment, mutated CTB or other non-ADP ribosylating subunits of toxins are used. Examples of such mutations include any point mutation, deletion or insertion into these toxins, subunits or other proteins as well as any peptide extensions to these proteins whether placed in the amino-end, the carboxy-end or elsewhere in the protein and irrespective of whether these peptides have immunological properties by being B cell epitopes, T cell epitopes or otherwise stimulating or deviating the immune response. For example, a number of such mutants have been described in the literature (U.S. Patent No. 6,033,673 to Clements; Backström et al; Gene 1995; 165: 163-171; Backström et al., Gene 1996; 169: 211-217; Schödel et al., Gene 1991; 99: 255-259; Dertzbaugh et al., Infect. Immun. 1990; 58: 70-79).

In practicing the present invention, the allergen and the cell-binding B subunit may be linked to each other directly or indirectly or merely mixed together. In the case where they are linked, the linkage may be covalent or non-covalent (*e*.*g*., via electrostatic and/or hydrophobic interactions).

In one embodiment, direct linkage between the allergen and cell-binding molecule (CTB) is achieved by chemically or genetically cross-linking the allergen and the cell-binding moiety CTB. Methods of conjugating proteins or peptides to carrier antigens and generating fusion proteins are well-known in the art (detailed protocols are described in Rask et al., Clin. Exp. Allergy 2000, 30:1024-1032 and Schödel et al., Gene 1991; 99:255-59. It will be understood that any suitable method may be used to cross-link the components, as long as the final cross-linked product retains the ability to induce suppression of the allergic response to the specific antigen/allergen employed. Suitable chemical cross-linking procedures are well known in the art; see, for example, Carlsson J. et al., Biochem. J. 173:723-737, 1978; Cumber, J.A. et al. Methods in Enzymology 112:207-224, 1985; Walden, P. et al., J. Mol. Cell Immunol. 2:191-197, 1986; Gordon, R.D. et al., Proc. Natl. Acad. Sci. (USA) 84:308-312, 1987; Avrameas, S. et al., Immuno-chemistry 6:53, 1969; Joseph, K.C. et al., Proc. Natl. Acad. Sci. USA 75:2815-2819, 1978; Middlebrook, J.L. et al., Academic Press, New York, pp. 311-350, 1981).

Indirect linkage between the allergen and the B subunit may be achieved using a spacer molecule. Preferably, the spacer molecule has an affinity for either the allergen, the mucosa-binding molecule, or both. In one embodiment, the spacer comprises an antibody, preferably a bifunctional antibody that recognizes both the allergen and the mucosa-binding molecule. In another embodiment, the spacer molecule is derived from the cholera toxin-binding structure of the GM1 ganglioside, galactosyl-N-acetyl-galactosaminyl-(sialyl)-galactosylglucosylceramide.
In these cases, the linkages are formed by high-affinity binding between the spacer and the other components. The only requirement is that the allergen and B subunit both remain linked to each other via the spacer during mucosal administration. In the case of spacer molecules that do not have specific affinity for either allergen or B subunit, chemical cross-linking methods may be used as described above to form covalent linkages between the components. In another embodiment, an indirect linkage may be achieved by encapsulating the allergen within a protective vehicle such as a liposome (or equivalent biodegradable vesicle) or a microcapsule, on the surface of which the B subunit is arrayed. In this type of presentation form, the allergen may be free within the lumenal space of the vesicle or microcapsule, or may be bound to other components. The only requirement is that the allergen and B subunit remain in close enough proximity during administration such that the allergen is effectively delivered through the skin.

Pursuant to the present invention, an effective amount of purified allergen for use with CTB would range between about 1 µg to 1 mg, preferably about 100 µg/cm². An effective amount of pure CTB would range between about 5 and 250 µg per cm². Administration is variable according to the nature of the allergen(s) present in the composition. It would not be difficult to one skilled in the art to determine such concentrations using *e*.*g*., provocation tests, skin prick tests (see *e*.*g*., Rhodius et al., Ann Allergy Asthma Immunol. 2002;88(4):374-9), or determining specific IgE levels against the allergen (Pierson-Mullany et al., Clin Exp Allergy 2002;32(1):107-16). One would determine the dose tolerated by giving 1 dose weekly for 3-6 months or daily doses for 4 weeks. The dosage range of the CTB-allergen fusion protein will be dictated by the amount of active CTB *i*.*e*,. 5 to 250 µg per cm². The amount of allergen peptide fused to it which would be at ratio of 5 allergen peptide mole per one mole of CTB pentamer since one molecule of CTB is made of 5 monomers each being fused to one peptide. If the amount of peptide is found to be insufficient, it can be increases by preparing CTB fusion proteins comprising up to three peptide repeats per monomer *i*.*e*., 15 peptide per CTB pentamer.

### Adjuvants

The formulation for induction of an immune response according to the present invention may contain an adjuvant, although a single molecule may contain both adjuvant and antigen properties (*e*.*g*., cholera toxin) (Elson and Dartzbaugh, Infect Immun. 1993; 61(1):48-55.). Adjuvants are substances that are used to specifically or non-specifically potentiate an antigen-specific immune response. Usually, the adjuvant and the formulation are mixed prior to presentation of the antigen but, alternatively, they may be separately presented within a short interval of time. Adjuvants include, for example, an oil emulsion (*e*.*g*., complete or incomplete Freund's adjuvant), a chemokine (*e*.*g*., defensins 1 or 2, RANTES, MIP1-.α., MIP-2, IL-8) or a cytokine (*e*.*g*., interleukin-1β., IL-2, IL-6, IL-10 or IL-12; interferon-gamma (IFN-γ); tumor necrosis factor-.alpha (TNF-α); or granulocyte-monocyte colony stimulating factor (GM-CSF) (reviewed in Nohria and Rubin, 1994)), a muramyl dipeptide derivative (*e*.*g*., murabutide, threonyl-MDP or muramyl tripeptide), a heat shock protein or a derivative, a derivative of Leishmania major LeIF (Skeiky et al., J Exp Med. 1995;181(4):1527-37.), CTB, a lipopolysaccharide (LPS) derivative (*e*.*g*., lipid A or monophosphoryl lipid A), or superantigen (Saloga et al., J Invest Dermatol. 1996;106(5):982-8.).

In another aspect of the method according to the invention, said allergen is a derivative, analog or mutant of an allergen. In particular, said derivative is a fragment comprising one or more than one T cell epitope. More particularly; said derivative or analog comprises one or more epitope capable of eliciting an IgE response. Even more particularly, said derivative comprises at least one epitope capable of interacting with specific IgE antibodies. In that case, said derivative is chemically linked to cholera toxin B subunit or to *E. coli* heat-labile toxin B subunit. In another aspect of the method according to the invention, the non-ribosylating toxin comprises and aldehyde-treated toxin. In particular, said allergen and said non-ADP ribosylating toxin subunit are administered in a formulation containing an aldehyde.
More particularly, said non-ribosylating toxin is cholera toxin or heat-labile toxin from *E. coli.*

The present invention is described further below in working examples which are intended to further describe the invention without limiting its scope.

### EXAMPLES

The present invention is also described by means of examples, including the particular Examples presented here below. The use of such examples anywhere in the specification is illustrative only and in no way limits the scope and meaning of the invention or of any exemplified term. Likewise, the invention is not limited to any particular preferred embodiments described herein.

### Methods

The descriptions and references provided readily permit those of ordinary skill in the art to reproduce the experiments as well as to undertake variations and modifications both with regard to the nature and choice of antigens used, the methods of conjugation or genetic fusion, and the methods used for preparation of cells and immunological assays.

In the examples below the following materials and methods were used.

**Animals.** Female BALB/c mice were purchased from Charles River (L'Arbresle, France). In all experiments 6-8 wk-old female mice maintained on an OVA-free diet were used. Male Sprague-Dawley rats, 12-14 weeks old, were purchased from BK Universal (Stockholm, Sweden).TCR-HA transgenic mice expressing a TCR specific for the HA immunodominant epitope of influenza virus haemagglutinin (aa 111-119), restricted by MHC class II I-Ad were kindly provided by H. von Boehmer (Harvard University, Boston). TCR-OVA transgenic Balb/c mice (DO11) expressing a TCR specific for the immunodominant epitope spanning aminoacid 323 to amino acid 339 of OVA were kindly provided by N. Lycke (Göteborg University, Sweden).

**CTB and CTB-OVA conjugate preparation**. Recombinant CTB was produced by a mutant strain of *Vibrio cholerae* 0 1, deleted of its CT genes and transfected with a multicopy plasmid encoding CTB. The CTB used was purified from the culture medium by a combination of salt precipitation and chromatographic methods, as described (Lebens et al., Biotech., 11:1574-8, 1993 and Sanchez et al., Proc. Natl. Acad. Sci. USA, 86:481-5, 1989). Ovalbumin (OVA), grade V was obtained from Sigma (St Louis, MO). Purified cholera toxin A subunit (CTA) was obtained from List Biologicals (Campbell, CA) and contained negligible amounts of CTB (<0.3%) as determined by GM1-ELISA. OVA was conjugated to CTB using N-succinimidyl (3-(2-pyridyl)dithio)-propionate (SPDP, Pharmacia) as bifunctional reagent essentially according to the supplier's instructions. Briefly, CTB and OVA were separately derivatised with SPDP at molar ratios 1:4 and 1:1.5, respectively, after which the OVA-SPDP was reduced and mixed with CTB-SPDP. The resulting conjugate retained both GM1-binding activity and CTB and OVA serological reactivies, as judged by a solid-phase ELISA using GM1 as capture system (Svennerholm et al., Curr. Microbiol. 1978; 1:19-23) and antibodies to CTB and OVA as detection reagent.

**Construction and purification of CTB fusion proteins.** Methods have been described for making fusion proteins based on CTB or LTB wherein nucleic acids encoding for either or both of T or B epitopes of an antigen of interest are genetically fused to coding sequences for either or both of the N- or C-terminus of CTB, or placed in an intrachain position in the CTB or LTB coding sequence, or to analogous positions in CTA or LTA (Bäckström et al., Gene 1995; 165:163-171, Bäckström et al., Gene 1994; 149:211-217, Schödel et al., Gene 1991; 99:255-259). Methods have also been described for fusing peptides to the carboxy or amino ends of CTA or LTA and for co-expressing these fusion proteins with CTB or LTB (Sanchez et al. FEBS Lett. 1986; 208:194-198, Sanchez et al. FEBS Lett. 1997; 401:95-97).

Two recombinant CTB fusion molecules are described in U.S. provisional application Serial No. 60/290,732. In one fusion molecule, peptide 323-339 of OVA was fused to the C terminus of CTB (CTB::OVAp), and in another, a peptide corresponding to the influenza virus haemagglutinin residues 108-119 (HA) replaced residues 56-63 in the CTB structure (CTB56 63Hap.

CTB::OVAp: Synthetic oligonucleotides encoding the 17 amino acid OVA peptide were synthesised (Innovagen AB, Lund, Sweden). The oligonucleotides were annealed and then ligated onto the 3' end of a CTB gene. DNA sequencing of the final plasmid, pML CTB::OVA, confirmed the final sequence of the gene fusion.

CTB56 63HAp: Synthetic oligonucleotides encoding the 12 amino acids HA peptide were synthesised (Innovagen AB). The oligonucleotides were inserted into plasmid pCB56 63gp12 (Bäckström et al., Gene 1995; 165:163-171) between positions 55 and 64 of mature CTB. DNA sequencing was used to confirm the sequence of the insert in pCB56 64HA.

Recombinant proteins carrying peptides inserted into CTB are more stable than those carrying peptides linked to the N or C termini (Bäckström et al., Gene 1994 149:211-217). Thus, whereas CTB56 63HAp could be expressed in Vibrio cholerae, CTB::OVAp was expressed in E. coli to avoid cleavage by extracellular V. cholerae proteases known to readily destroy C terminal peptide extensions on CTB (Schödel et al., Gene 1991; 99:255-259).

pCB56 63HAp was transferred into V. cholerae strain JS1569 by electroporation (Lebens et al., Bio Technology 1993; 11:1574-1578). The protein was precipitated from the growth medium using hexametaphosphate (Lebens et al. Bio Technology 1993; 11:1574-1578), and was then re-dissolved in a minimal volume of 0.2 M Tris HCl pH 8.0 and dialysed against PBS pH 7.2.

pML CTB::OVA was transferred into *E. coli* BL21 (Grodberg et al. J. Bacteriol. 1998; 170:1245-1253). The CTB gene used lacked the signal peptide directing transport of the synthesised protein into the periplasmic space. This resulted in the cytoplasmic accumulation of the product (CTB::OVAp) as monomers, which formed insoluble inclusion bodies. These were dissolved in 6.5 M urea and reassembled by dialysis (L'Hoir et al, Gene 1990; 89:47-52).

The CTB::OVAp and CTB56 64HAp fusion proteins were further purified by ion exchange (Resource Q column, Pharmacia Biotech, Uppsala, Sweden) and FPLC gel filtration (Superdex 200 16/60 column, Pharmacia Biotech of Uppsala, Sweden) using the Biologic Workstation FPLC system (Biorad of CA, USA).

The CTB fusion proteins were shown to have retained GM1 binding activity by means of GM1 ELISA using biotinylated anti CTB monoclonal antibodies.

**Immunizations and preparation of samples**. For the epicutaneous immunizations, Balb/c mice were shaved on the abdomen with a surgical blade 24 hours prior to immunization. The animals were then anesthetized during one hour by i.p. injection of sodium pentobarbital (60 mg/g mouse) (Sanofi), and 200 µl of the immunizing solution was placed on the shaved skin over a 4 cm2 surface area and left for one hour. Following the incubation, the mice were extensively washed with tap water. Mice received 3 consecutive epicutaneous immunizations at days 1, 14, and 28. Mice were also immunized by subcutaneous (s.c.) injection in one footpad with 100 µg OVA emulsified in Freund's complete adjuvant (CFA) or in other experiments Balb/c mice were sensitized and challenged intraperitoneally (i.p.) with 10 mg OVA adsorbed on 2 µg of Al(OH)₃ (Sigma). Sera were prepared from tail vein blood from pre- and post- immune mice.

**Induction of IgE antibody responses.** Balb/c mice were sensitized and challenged intraperitoneally (i.p) with 100 µg OVA adsorbed on 2 mg A1(OH)3 gel (alum, Superfos Biosector, Kvistgaard, Denmark) at time-points indicated in each figure and in Table 1on day 0 and 14. Sera were prepared from tail vein blood.

**Measurement of serum IgG levels.** Serum antibodies against CTB and OVA were determined using enzyme-linked immunosorbent assay (ELISA). Briefly, high binding 96-well polystyrene plates (Greiner) were coated with either CTB (0.1 mg in 100 ml) or OVA (1 mg in 100 ml) overnight at room temperature (RT) followed by a step of saturation of 90 minutes with 20 mg/ml BSA. All steps were performed at room temperature and followed by three washes with PBS-Tween (0.05%). Sera were serially diluted in 2 mg/ml BSA and incubated in the coated wells for 3 hours. After washing, the plates were incubated during 1 hour with HRP-labeled goat anti-mouse total IgG (Southern Biotechnology Associates) diluted in PBS-Tween, and solid-phase bound HRP activity monitored after addition of chromogenic substrate consisting of OPD and H2O2, and the absorbance at 450 nm was measured spectrophotometrically. For detection of specific serum IgG1, IgG2a, and IgG2b antibody levels to OVA, 96-well low-binding polystyrene plates (Nunc, Roskilde, Denmark) were coated with OVA (10 µg/ml 0.1 M NaHCO3, overnight at RT). Serially diluted mouse sera were incubated for 1 h and the plates were washed with PBS. Solid phase-bound antibodies were detected as above by stepwise incubation with peroxidase-labeled goat antibodies to mouse IgG or IgG1, IgG2a, or IgG2b (Southern Biotechnology) and finally developed with enzyme substrate. The titer of a serum was defined as the reverse of the highest dilution yielding an absorbance value of 0.4 above background. Data are expressed as geometric mean titers +/- standard deviation in different treatment groups. The non-parametric Wilcoxon Mann-Withney's rank test was used to compare experimental groups for statistical differences.

**Measurement of serum IgE levels.** Total concentrations of serum IgE were determined by a solid phase sandwich ELISA. Briefly, 96-well high-binding polystyrene plates (Greiner, Labtechinik, Frickenhausen, Germany) were coated over night at room temperature (RT) with a rat monoclonal antibody to mouse IgE (Pharmingen, San Diego, CA) diluted to 2 µg/ml in PBS. Serially diluted mouse sera were incubated for 2 h at RT. After washing with PBS, biotinylated rat monoclonal anti-mouse IgE (Southern Biotechnology) antibody reagent was added and incubated for 1 h at RT. Finally, peroxidase-conjugated avidin (Sigma, S Lois, MO, USA) was added for 1 h at RT. The plates were washed and developed with H2O2 and orthophenylene diamine (OPD) (Sigma) and the absorbance at 450 nm was measured. The concentration of total IgE was calculated by extrapolation from a standard curve generated by assaying known amounts of a purified mouse IgE (Pharmingen).

**Lymphocyte proliferation.** OVA-specific proliferative responses were determined on triplicate cultures of DLN cell suspensions obtained from immunized or control mice. Cells were seeded at 4 x 10⁵ cells per flat-bottom well of 96-well-culture plates. After incubation at 37°C for 72 hours in the presence or absence of OVA (2mg/ml), cultures were pulsed for another 18 hour period with 1 µCi of [³H]thymidine. Alternatively, 10⁴ MLN DCs isolated from DLNs of mice epicutaneously treated with CT or CTB or from control mice were cultured for 72 h with 2 x 10⁵ transgenic TCR-HA T cells in the presence or absence of dodecapeptide HA (aa 111-119 from influenza haemagglutinin) and pulsed with 1mCi of [³H]thymidine during the last 18h of culture. Cultures were harvested onto glass filters using a cell harvester and the extent of radioactive thymidine incorporated was measured with a beta scintillation counter.

**Cytokine assays.** Culture supernatants from DLNs after *in vitro* stimulation with OVA were assayed for IL-2, IFN-γ, IL-4 and IL-5 contents by ELISA using R&D duosets according to manufacturer's instructions. Briefly, high binding 96-well polystyrene plates (Nunc, Roskilde, Denmark) were coated with 100 µl of either anti-IL-2, IFN-γ, IL-4 and IL-5 coating antibodies (2 µg/ml) in 0.1 M phosphate buffer overnight at RT followed by a step of saturation of 60 minutes in 1% non fat milk powder with 0.12% Triton X 100. All steps were performed at RT and followed by three washes with PBS containing 0.05% Tween 20 (PBST). Supernatants were diluted 2- to 5-fold in PBST with 1% non fat milk and incubated for 2h at RT. After washing, the wells were exposed for 1 hour to biotinylated antibodies to IL-2, IFN-γ, IL-4 or IL-5 (0.1 µg/ml) in PBST, and developed by stepwise addition of streptavidin-biotinylated horseradish peroxidase complex (ABC complex, Amersharn) and 3,3',5,5'-tetramethyl benzidine (TMB)/H2O2 (Kirkegaard and Perry laboratories, Gaitherburg, MA).

**Passive cutaneous anaphylaxis (PCA) titrations**. The levels of circulating anti-OVA IgE antibody were assayed by PCA (Ovary, Z., In: Ackroyd, J.F. eds. Immunological Methods. Oxford: Blackwell Scientific Publications, 1964:259-84 and Watanabe, N and Ovary, Z., J. Immunol. Meth., 14:381-90, 1977). In brief, Sprague-Dawley rats were anesthetized by i.p. injections of Hypnorm^{®} (0.032 mg fetanyl citrate and 1 mg fluanisone per rat) and Stesolid Novum® (0.5 mg/rat). Test mouse sera were serially two-fold diluted in PBS, and 50 ml of each dilution was injected intradermally into the shaved dorsal skin of rats. Seventy-two hours later the rats were challenged intravenously with 5 mg OVA diluted in 1 ml pyrogen-free PBS containing 1 % Evans blue (Sigma). One hour later, the rats were sacrificed and the skin areas were examined for the appearance of blue reactions. The PCA titers were defined as the reciprocal of the highest dilution of serum giving a blue spot larger than 5 mm in diameter.

A characteristic of the IgE antibody class is that heat treatment (56°C, 4 h) destroys its skin-sensitizing ability (Ovary, Z., J. Immunol. Meth., 14:381-90, 1977). Heat treatment (56°C for 30 min) of randomly-selected sera from saline fed OVA-sensitized and OVA-challenged animals virtually abrogated the PCA activity of corresponding sera, indicating that the PCA activity measured was mediated almost exclusively by IgE antibodies.

**Preparation of epidermal LCs and DLN DCs**. Ears from mice were rinsed with 70% ethanol, split with the aid of forceps into dorsal and ventral halves, and incubated in PBS containing 5% FCS in the presence of 20 mM EDTA for 45 min at 37° C, to allow the separation of the epidermis from the dermis. Epidermal sheets were then put onto glass slides and dried. Alternatively, dorsal and ventral halves were incubated with 0.5% trypsin (Sigma, St. Louis, MO) in PBS containing 5% FCS for 45 min at 37°C, to allow the separation of the epidermis from the dermis. Epidermal cell suspension was then obtained by mechanical extraction of cells from the separated epidermis and LCs represented 0.5 to 4% of total cells.

DC were purified from DLNs, following an isolation protocol previously described (Anjuere et al., Dendritic cell protocols. 2001; vol. 64. Humana Press Inc., Tolowa, NJ; Anjuere et al., Blood. 1999; 93:590-598). Briefly, DLNs were digested with collagenase A (0.5mg/ml; Boehringer-Mannheim, Mannheim, Germany) and DNase I (0.4mg/ml, Boehringer-Mannheim) in RPMI 1640 medium supplemented with 5% FCS for 10 min. at 37°C with continuous agitation. Digested fragments were filtered through a stainless-steel sieve, and after washes, cell suspensions were then resuspended in cold isoosmotic OptiprepTM solution (Nyegaard Diagnostics, Oslo, Norway), pH 7.2, density 1.061 g/cm3. A low-density fraction, accounting approximately for 1% of the starting cell population, was obtained by centrifugation at 1700xg for 10 min. T-lineage cells, B cells, macrophages and granulocytes were then depleted by treating the recovered low-density cells for 40 min at 4°C with a mAb mixture including anti-CD3 (clone KT3-1.1), anti-macrophage antigen F4/80 (clone C1.A3-1), anti-BB20 (clone RA3-6B2) and anti-granulocyte antigen Gr1 (clone RB6-8C5). The unwanted cells were removed magnetically after incubation for 30 min at 4°C with a 1:1 mixture of anti-mouse Ig and anti-rat Ig coated magnetic beads, (Dynabeads, Dynal, Oslo, Norway) at a 7:1 bead-to-cell ratio. DC preparations obtained by this technique were >85% pure.

***In vivo* depletion of LCs by UV-C irradiation**. Mice were anesthetized with pentobarbital, and placed with their ears ca 30 cm from a UV-C source (G40T10 UV Lamp) and irradiated during 45 minutes (total irradiation: 80 mJ/cm²). LC depletion was confirmed by MHCII staining of both epidermal sheets and epidermal cell suspension.

**Preparation of paraffin-embedded ear transverse sections**. Mice were sacrificed at various times after epicutaneous treatment with CT or CTB and their ears were excised. The ears were then embedded in low melting point paraffin using a technique allowing preservation of antigens (Becksteadt et al., J. Histochem. Cytochem. 1994; 42:1127-1134). Briefly, the ears were fixed overnight at 4°C in a Tris-calcium acetate buffer containing zinc acetate (0.5%) and zinc chloride (0.5%) and dehydrated in solutions of acetone (70% and 100%). Specimens were then embedded in low melting point paraffin wax (BDH laboratory, melting point 37-39°C). Serial 10-µm sections were prepared and transferred to glass microscope slides (Starfrost). Slides were dried overnight at room temperature and stored desiccated at 4°C.

**Immunohistochemistry.** The slides were de-waxed in acetone for 3 minutes and washed with PBS. The sections were circled with a waterproof pen (Dako). Non-specific binding sites were blocked with 10% fetal calf serum (FSC) diluted in PBS for 30 minutes at RT. Sections were then incubated with biotin-conjugated anti-MHCII monoclonal antibody (clone 2G9, Pharmingen, San Diego, CA) (1 µg/ml) or with an isotype control antibody (clone B39-4, Pharmingen) at the same concentration during one hour at RT. Specific staining was revealed after incubation during 30 minutes with ABC-HRP complex (Amersham) followed by incubation with a chromogen substrate (H2O2 and 3-amino-9-ethylcarbazole; H2O2-AEC). Slides were counterstained with hematoxylin-eosin.

**Flow cytometry.** Phenotypic analysis of DC subpopulations was performed after triple staining with fluorescein (FITC)-conjugated anti-CD11c (clone N418, prepared in the laboratory), phycoerythrin (PE)-conjugated anti-CD8a (clone 53-6-72, Pharmingen) and biotin-conjugated anti-CD11b (clone M1/70, Pharmingen), anti-B7-1 (clone 16-10A1, Pharmingen), anti-B7-2 (clone GL1, Pharmingen), anti-CD40 (clone 3/23, Pharmingen) followed by streptavidin-tricolor (Caltag, San Francisco, CA). Analysis of LCs was performed after double staining with PE-conjugated anti-MHCII (clone 2G9, Pharmingen) and Fluorescein-conjugated anti-CD11c, biotin-conjugated anti-B7-1, biotin-conjugated anti-B7-2, biotin-conjugated anti-CD40, followed by streptavidin-tricolor. All the staining steps were performed at 0-4°C in PBS containing 5 mM EDTA and 2% FCS. Analysis was performed on a FACScan flow cytometer (Becton Dickinson & Co., Mountain View, CA).

**Statistics.** Data were compared by nonparametric Mann - Whitney or Fisher's exact test and two-tailed P-values <0.05 were considered significant.

### Example 1: Both CT and CTB Transcutaneously Induce Strong Serum Antibody Responses Against Themselves

When given topically onto the skin, CTB was bound to be as efficient as CT to induce the production of specific antibodies to itself (Figure 1A). After three epicutaneous treatments, all mice had responded with anti-CTB antibody titers at or above 10,000.

### Example 2: CTB is Less Efficient Than CT in Promoting a Systemic Antibody Response to Co-Administered OVA

The ability of CTB, as compared to CT, to promote systemic antibody responses to a prototype co-administered antigen (OVA) was evaluated. As shown in figure 1B, the topical application of OVA with CTB led to the production of significant anti-OVA IgG (geometric mean titer=720) whereas OVA administered onto skin was not able to induce the production of IgG against itself (titers at or below 10). However, the magnitude of the anti-OVA response induced by OVA co-administered with CTB was fivefold lower than that evoked by OVA together with CT (geometric mean titer=3500) (Figure 1B). Even so, also the latter levels of anti-OVA specific antibodies were lower than the level induced by an i.p. injection of OVA in alum (Figure 1B).

### Example 3: Co-Administration of Either CT or CTB With OVA Leads to Vigorous OVA-Specific T Cell Proliferative Responses Both in Draining Lymph Nodes and at Distant Systemic Sites

The specific proliferative responses in cells from lymph nodes draining the epicutaneous site of immunization (DLN) and from the spleen was evaluated. For this, the OVA-induced proliferation of such cells was measured during *in vitro* restimulation with 2mg/ml OVA after three consecutive epicutaneous immunizations.

Topical application of OVA with CT or CTB led to the generation of similarly strong anti-OVA proliferative responses in DLN cells (Figure 2A). These responses were specific for OVA as indicated by the lack of any *in vitro* response to an unrelated protein antigen, LACK (not shown). Interestingly, epicutaneous treatments with OVA and CT or CTB were as efficient in inducing anti-OVA proliferative responses as subcutaneous injection of OVA in CFA, a treatment known to be very potent in inducing cellular responses. Furthermore, TCJ with OVA in presence of either CT or CTB led to the generation of similar, strong anti-OVA proliferative responses also in the spleen (Figure 2B). Comparable OVA-specific proliferative responses were also seen after topical application of a CTB-OVA conjugate, showing that the transcutaneous transport of OVA was not dependent on covalent coupling of OVA to CTB.

### Example 4: CTB Suppresses Epicutaneously Induced IgE Responses To Oyabulmin

Mice were immunized epicutaneously with either free OVA alone or OVA co-administered with 50 µg of either CT or CTB every two days during two weeks. Three weeks after the last TCI, the animals were sensitised systemically with alum-adsorbed OVA. Sera were collected three weeks after sensitization and their anti-OVA reaginic antibody titers were determined by PCA in rat recipients. As shown in Figure 4A, whereas epicutaneous CT potentiated high anti-OVA IgE titers, epicutaneous treatment with CTB reduced the PCA titer as compared to control animals epicutaneously immunised with OVA alone.

To evaluate whether TCI with CTB suppresses allergen-specific IgE antibody responses in sensitized mice, mice were sensitized and challenged by i.p. injection of alum-adsorbed OVA on day 0 and week 14. Animals were treated by TCI with OVA alone or co-administered with either CT or CTB three times a week for 4 consecutive weeks starting on week 12 and until week 16, i.e. two weeks before and after booster injection. PCA titers were determined immediately before and three weeks after the OVA booster injection. As shown in Figure 4B, TCI with OVA and CTB significantly suppressed IgE responses in most animals (6/7) in comparison with control mice (p<0.02) whereas CT failed did not suppress IgE responses, and to the contrary, increased IgE titers.

### Example 5: CTB Selectively Potentiates Th1-Driven-Responses, and Increased IL-2 and IFN-γ Production Without Affecting Th2-Dependent Responses

We analyzed the cytokines produced by DLN or spleen cells after three consecutive TCIs with either OVA alone or OVA together with CT or CTB, after *in vitro* restimulation with OVA. As shown in Figure 3 and Table 1, CTB potentiated selectively the production of IL-2 and IFN-γ by spleen cells, and to a lesser extent by DLN cells, from immunized mice in comparison with control mice. Such treatment on the other hand did not increase *in vitro* OVA-induced IL-4 and IL-5 responses. By contrast, the co-administration of antigen with CTB on the other hand did not induce levels of IL-4 and IL-5 different from those induced by OVA alone (or unimmunized control T cells) in DLN.

In contrast, CT potentiated the production of both Th-1 (IFN-γ) and Th-2 (IL-4, IL-5) cytokine by spleen cells (Table 1). Similar results were obtained with DLN cells (Figure 3). Moreover, TCI with a mix of CTA and CTB induced levels of Th1 (IFN-γ) and Th2 (IL-4 and IL-5) cytokines comparable to those induced with CT. Interestingly, TCI with OVA and CTA, a treatment that failed to induce detectable antibody responses and proliferative responses to OVA selectively enhanced the production of Th2 cytokines (IL-4 and I-5) (Table 1), indicating that CTA may penetrate and/or facilitate the passage of free OVA through the skin.

**Table 1**

| | **IFN-γ** | **IL-4** | **IL-5** |
|---|---|---|---|
| **TCI** | | **(pg/ml)** | |
| CT+OVA x3 | 236 ± 30 | 27 ± 1 | 54 ± 40 |
| CTB+OVA x3 | 120 ± 60 | 2 ± 1 | <10 |
| CTA+CTB+OVA x3 | 396 ± 200 | 28 ± 4 | 102 ± 106 |
| CTA+OVA x3 | 51 ± 8 | 29 ± 2 | 109 ± 2 |
| CFA+OVA x 1 | 256 ± 30 | <10 | <10 |

Data in Table 1 are expressed as arithmetic mean levels of cytokines determined on triplicate cultures of spleen cells exposed for 48h to OVA and are representative of 3 experiments.

### Example 6: Epicutaneous CT and CTB Treatments Increased LC Numbers in the Epidermis

The topical application of CT and CTB onto ear skin affected the density of LC in the epidermis was tested. Epidermal sheets from the ears of mice excised 90 minutes after epicutaneous treatment with CT and CTB and assessed LC numbers in the sheets by counting MHCII+ cells after enzymatic immunohistochemical staining was prepared. As shown in Figure 5A, epicutaneous treatment with 20 µg of CT or CTB led to a 20% and 50% increase in LC numbers compared to control epidermal sheets.

Furthermore, since the turnover of LCs in the epidermis is known to be slow we also induced a partial egress of resident LCs before epicutaneous treatment by exposing the skin to UV-C irradiation which has been previously described to be the most effective UV treatment in promoting LC depletion (Anjuere et al, Blood 2000; 96:1633-1637; Nithiuthai, Immunology. 1984; 51:143-156). Thus, 36 hours after UV-C irradiation for 45 minutes, mice were treated with CT or CTB and then determined LC numbers in the epidermis in comparison LC numbers in buffer-treated control mice. MHCII+ LC in both epidermal sheets after immunohischemical staining and in epidermal cell suspensions after staining with fluorescent antibodies specific for MHCII and CD11c molecules were counted, followed by flow cytometry analysis. As shown in Figure 5B, UVC irradiation induced a 50% depletion in LC, and CT and CTB treatment led to the recruitment of threefold and twofold more LC respectively, as compared with a control. Similar differences in LC density were observed in epidermal sheets (data not shown). Furthermore, after MHCII staining, epidermal sheets of CT-and CTB-treated mice presented numerous clusters of MHCII+ LC cells (data not shown).

In order to evaluate the kinetics of recruitment of LC induced by the topical application of CT and CTB, skin transverse sections 20, 40, 90 and 180 minutes after epicutaneous treatment with either CT and CTB were prepared, and LC frequency and localisation by staining with a monoclonal antibody specific for MHCII, CD11c and DEC-205 followed by immunoenzymatic detection was analyzed. As shown in Figure 5C, in a control mouse we observed scattered staining with anti-MHCII antibody both in the dermis and epidermis. As early as 40 minutes after treatment with either CT or CTB, an accumulation of MHCII+ cells organized in clusters and localized in particular at the dermo-epidermal junction of ear transverse sections, but also more numerous MHCII+ cells in the epidermis as compared with control mice were found. This accumulation was even more intense three hours following the epicutaneous application of CT or CTB and persisted for at least five hours (Table 2). Results are expressed as the percent increase in LC numbers in CT and CTB-treated mice compared to control mice treated with diluent (PBS) alone. Furthermore, flow cytometry analyses of CD11c+, MHC class II+ cells from draining lymph nodes disclosed that whereas CT induced an increase in DC numbers in lymph nodes, CTB failed to do so (Table 2). Such increase seen with CT was maximal 24 hrs after treatment and progressively decreased by 48 hrs.

**Table 2**

| **Treatment** | **Skin DC** **(Langerhans cells)** | **Lymph node DCs** |
|---|---|---|
| | **90 min** | **24 hrs** |
| CT | 220% | 180% |
| CTB | 330% | -20% |
| PBS | 0% | 0% |

### Example 7: Epicutaneous CT and CTB Treatments Differentially Modified the Maturation Status of LC in the Epidermis

The maturation status of LC was evaluated by analysing the levels of expression of co-stimulatory molecules B7-1, B7-2 and CD40 as well as MHCII molecules by flow cytometry after staining of LC suspension, performed as previously described with fluorescent monoclonal antibodies specific for these molecules. As shown in Figure 6A, 90 minutes after topical application of CT and CTB, a twofold decrease of B7-2 expression and a threefold decrease in CD40 and B7-1 expression by LC was observed as compared with control LC. Thus, the rapid increase in LC density in skin after CT and CTB epicutaneous treatments may correlate with a recruitment of less mature immigrants in the epidermis. Furthermore, 20 hours after epicutaneous treatment with CT, we observed a twofold increase in B7-1 expression by LC (Figure 6B), whereas CTB did not induce any up-regulation of B7-1, B7-2 or CD40 molecules. MHCII expression was not significantly affected in any instance (data not shown).

### Example 8: Epicutaneous CT and CTB Treatments Affected the Immunostimulatory Properties of DC In Vitro

It has been extensively described that LC migrate to draining lymph nodes (DLN) where they as mature DC are able to induce immune responses. In order to investigate the immunomodulatory effects of topical application of CT and CTB onto skin in draining lymph nodes, we tested the *in vitro* immunostimulatory properties of draining lymph node DC (DLN DC) from mice epicutaneously treated with CT or CTB 24 hours earlier or from control mice was tested. The ability of DLN DC to induce the proliferation of naive transgenic TCR-HA T cells *in vitro* in a 4-day antigen-dependent proliferation assay was assessed. As shown in Figure 7, DLN DCs isolated from mice epicutaneously treated with CT and CTB were 50% and 25%, respectively, more efficient at inducing proliferation of naive T cells than were DCs isolated from PBS-treated control mice.

The expression of costimulatory molecules B7-1, B7-2 and CD40 by DLN DC subsets from mice CT-and CTB-treated mice was then analyzed. As shown in figure 8A, three distinct subsets of DC were found which were formed CD8+, CD8int and CD8- on the basis of the differential expression of CD8a and CD11b in peripheral lymph nodes draining the epicutaneous site of immunization (inguinal, axillar and auricular draining lymph nodes). CD8+ DC, CD8int DC and CD8- DC represent 5%, 76 % and 16% of total DC, respectively. As shown in Figure 8B, all three DC subsets of CT-treated mice up-regulated the expression of B7-2 costimulatory molecule by a factor of 1.6, but down-regulated slightly their CD40 expression as compared with control and CTB-treated DC (Figure 8B). In contrast, CTB did not induce any detectable change in the expression of B7-2 or CD40 by any of the three DLN DC subsets, but induced a twofold increase in B7-1 expression exclusively by CD8+ DC. In all cases, the levels of expression of B7-1, B7-2 and CD40 were higher than those expressed by LC (Figure 6), which is consistent with their more mature state.

### Example 9: Transcutaneous Administration of an Allergen-Derived Peptide Fused with CTB Suppresses IgE Responses to the Native Allergen OVA More than when Admixed with CTB

Groups of 6-8 Balb/c mice were treated with 50 µg of CTB mixed with 10 µg of OVAp or 60 µg of CTB:OVAp fusion protein given by topical skin application on 5 consecutive occasions on day 0, 1 2, 3 and 4. Five days after the last application (day 10), mice were injected intraperiteonally with 10 µg OVA protein in alum. For control purposes, additional groups of mice were treated with an irrelevant fusion protein (CTB:HAp), OVA peptide alone, CTB alone, or vehicle (PBS) alone. PCA titers were determined immediately immediately before and three weeks after the OVA/alum injection.

As shown in Table 3, treatment with OVA peptide mixed with CTB or conjugated to CTB as gene fusion protein was able to suppress IgE antibody responses. Treatment with OVA peptide alone or with an irrelevant gene fusion protein failed to inhibit such responses. Thus, treatment with a short peptide derived from the sequence of the allergen OVA and given onto skin either in a free form mixed with CTB or linked to CTB was able to suppress the allergic response induced by the systemic injection of the native allergen.

**Table 3**

| **Transcutaneous treatment:** | **PCA titer** | **% suppression** |
|---|---|---|
| CTB + OVA peptide | 120 ± 40 | 45% |
| CTB:OVAp gene fusion protein | 90 ± 60 | 60% |
| CTB:HAp gene fusion protein | 260 ± 100 | 0% |
| OVA peptide | 350 ± 110 | 0% |
| CTB | 330 ± 80 | 0% |
| Control PBS | 220 ± 60 | 0% |

### Example 10: Aldehyde-modified Cholera Toxin Loses Toxicity and Acquires Suppressive Properties of IgE Antibody Responses

Treatment of cholera toxin with glutaraldehyde: cholera toxin (1 mg) (LIST Biologicals) was incubated for 2 hrs with 0.05, 0.2, or 0.5% (final concentration) glutaraldehyde (Sigma Chemicals, St Louis, MO) in 0.01 M phosphate buffer, pH 6.8. The protein was then dialyzed overnight at 4°C against 0.1 M NaHCO3 buffer, pH 8.

Treatment of cholera toxin with formaldehyde: cholera toxin (1 mg) was incubated for one hour 4°C with 0.1, 0.5, or 2.5% paraformaldehyde (Aldrich Chemicals), and stored frozen until use.

For determination of GM1-binding activity, aldehyde modified CT preparations were assayed by means of a solid-phase GM1 ELISA using biotinylated antibodies to LTB.

For determinations of toxicity, aldehyde-modified CT preparations were dialysed overnight at 4 °C against pyrogen-free saline and 20 microliters of saline containing 10 microgram of each preparation was injected in the right rear footpad of Balb/c mice. After 4 hrs, the swelling response was determined with a calliper and compared to that of mice injected with 1 microgram of unmodified cholera toxin. Data were expressed as percent of the footpad swelling of cholera toxin-treated animals.

As shown in Table 4, treatment of CT with glutaraldehyde abrogated in a dose-dependent manner the toxicity of the toxin. At a concentration of 0.2%, the toxicity had essentially vanished while the toxoid retained GM1-binding activity. Similarly, treatment of CT with formaldehyde at concentrations of 0.5% or higher had similar effects.

**Table 4**

| **Treatment** | **Toxicity** | **%GM1 binding activity** |
|---|---|---|
| Nil | 100 | 100 |
| Glutaraldehyde 0.05% | 50 | 150 |
| Glutaraldehyde 0.2% | 0 | 70 |
| Glutaraldehyde 0.5% | 0 | 80 |
| Formaldehyde 0.1% | 70 | 110 |
| Formaldehyde 0.5% | 0 | 130 |
| Formaldehyde 2.5%% | 0 | 60 |

To evaluate whether CT modified with glutaraldehyde or with formaldehyde was able to suppress allergic responses to OVA, mice were exposed epicutaneously to 3 consecutive doses of CT (50 microgram per dose) modified with either 0.2% glutaraldehyde or 0.5% formaldehyde together with OVA on day 0, 4, and 10. On day 14, mice were injected alum-adsorbed OVA intra-peritoneally so as to elicit an IgE response. In animals treated with glutaraldehyde- or formaldehyde-modified CT, IgE responses were suppressed, whereas in animals similarly treated but with native CT, these responses were even increased as compared to sham-immunized mice (Table 5).

**Table 5**

| **Transcutaneous treatment** | **PCA titer** | **% suppression compared to control PBS** |
|---|---|---|
| PBS control | 220 ± 60 | --- |
| CT | 540 ± 120 | - 59% |
| 0.2% glutaraldehyde CT | 95 ± 55 | 56% |
| 0.5% formaldehyde CT | 70 ± 30 | 68% |

In view of the above, the present invention also contemplates a formulation comprising the allergen, a non-ADP-ribosylating toxin or subunit, and an aldehyde, for topical administration.

### Discussion

Described herein are experiments which demonstrate that both CT holotoxin and CTB, the non-toxic receptor-binding moiety of CT, when applied on the skin can penetrate the skin barrier and, importantly, can also facilitate the passage of heterologous and/or by other means enhance the immune response to a co-administered, either admixed or linked heterologous antigen. Therefore, when applied epicutaneously both CT and CTB induce an immune response against themselves and also against the co-administered heterologous antigen. However, in contrast to CT, which promoted both strong humoral and cellular responses to the heterologous antigens, TCI with an antigen applied together with CTB favors the development of vigorous systemic Th1 cellular response, but is only marginally efficient at promoting systemic antibody responses to a prototype protein antigen. Surprisingly, TCI with an antigen applied together with CTB was able to suppress an established, IgE-mediated, allergic immune response, while TCI with an antigen applied together with CT, enhanced the allergic immune response.

These data confirm recent findings indicating that CT can serve as an effective adjuvant for inducing humoral and cellular immune responses to various soluble protein antigens when co-administered onto the skin (Glenn et al., Nature 1998; 391:85; Glenn et al, Immun. 1999; 67:1100-1106; and Glenn et al., Nature Med. 2000; 6:1403-1406). This work also indicates that the ADP-ribosylating activity of the holotoxin does not appear to be critical for allowing the transfer of either itself or co-administered protein antigens through the epidermis, but may be responsible for enhancing the allergic response. The ability of the toxin and its B subunit to bind to GM1 receptors could thus be the most critical factor involved in the transport of these molecules through the epidermis.

The observation that the systemic IgG antibody responses generated after topical application of CT onto the skin were superior in magnitude to those seen with CTB, although of comparable isotype distribution, is not surprising. Indeed, CT is a powerful adjuvant when administered by a mucosal (Elson et al., J. Immunol. 1984; 133:2892-2897; Lycke et al., Immunology 1986; 59:301-308) or a systemic (Holmgren et al., Immunol Commun 1976; 737-756) route while CTB appears to be devoid of adjuvant activity when given by these routes. These observations support the notion that the adjuvant properties of CT or LT for humoral immune responses appear to be mainly associated with its enzymatic, ADP-ribosylating activity (Lycke, Eur. J. Immunol. 1992; 22:2277-2281).

Although CTB, when co-administered onto the skin could promote a modest systemic antibody response to a co-administered antigen, and then requiring repeated co-applications, it behaved as a surprisingly powerful and hitherto unknown adjuvant for cell-mediated immune responses. In this respect, the magnitude of proliferative responses induced by skin exposure to CTB and a prototype antigen was comparable to that induced by cutaneous exposure of the same antigen in the presence of CT. This CTB-induced response was also comparable to that seen after systemic injection of the same antigen in Freund's complete adjuvant, one of the most potent adjuvants known for inducing cellular immune responses. The divergent activities of CTB and CT on systemic humoral versus cell-mediated immune responses to antigens co-administered through the skin are consistent with the observation that CTB, like CT, enhances the production of Th1 cytokines but, in contrast to CT, does not affect Th2 cytokine production. These observations could also explain the results of both the present experiments (Fig. 5) and additional work showing that epicutaneous application of CT with a prototype allergen can sensitize mice for systemic IgE antibody responses (the archetype of Th2 responses) whereas CTB co-applied by the same route fails to induce such responses and in fact, suppresses these responses.

The observation that aldehyde treatment of whole CT inhibited toxicity but could maintain GM1 binding and was associated with anti-IgE activity upon skin co-application with allergen supports the notion that the toxic activity of native CT is associated with pro-Th2 activity.

The exceptional adjuvant activity of CT and CTB on cell-mediated immune responses to antigens co-applied onto the skin appears to be in part associated with their capacity to induce the rapid recruitment of DC into the exposed epidermis and their subsequent exit from the epithelium to the DLN. In this respect, exposure of the skin to CT or CTB was associated with a rapid increase in DC density in the epidermis and particularly in the underlying dermis. Furthermore, phenotypic analyses of DC isolated from epidermal sheets exposed to CT or CTB revealed increased frequencies of cells expressing MHC II and CD11c, with reduced expression of CD40, B7.1 and B7.2. This phenotype, characteristic of immature DC, suggests that the majority of DC having migrated to the epidermis after treatment with CT or CTB are mainly of dermal origin and could explain the overall decrease in the maturation characteristics of epidermal DC newly populated by immature DC.

The finding that treatment with CT, different from CTB, led to increased expression of B7.2 on dendritic cells from DLN and enhanced production of IL-4 and IL-5 is compatible with the notion that B7.2 is associated with preferential stimulation of CD4+ Th2 cells (Freeman et al., Immunity. 1995; 2:523-532).

It has been shown that, under the effect of different stimuli, keratinocytes secrete cytokines (granulocyte-macrophage stimulating factor/GM-CSF; TNF-α) and chemokines (MCP-1) that influence the migratory behavior and the differentiation of LC and DC (Bos et al., Immunol. Today. 1994;14:75-78). These data are consistent with preliminary studies indicating that skin epithelial cells from CT- and CTB-treated mice express increased levels of MIP-3α mRNA, a chemokine produced by keratinocytes (Nakayama et al., Int. Immunol. 1001; 13:95-103) that play a major role in the trafficking of epidermal LC (Charbonnier et al, J. Exp. Med. 1999; 190:1755-1768) and in the attraction ofLC precursors into the skin (Dieu-Nosjean et al., J. Exp. Med. 2000; 192:705-718).

As shown herein, the adjuvant properties of CT for Th1 and Th2-driven responses to involve distinct structural determinants associated with the B and A subunits, respectively. The fact that CTB suppresses systemic Th1 responses when given by a mucosal route but potentiates these responses when given by the epicutaneous route underscores the role of the epithelial microenvironment in the regulation of immunity.

**Appendix A**

| **ALLERGEN SOURCE** | **SYSTEMATIC AND ORIGINAL NAMES** | **MW KDA** | **SEQ** | **ACCESSION NO. OR REFERENCES** |
|---|---|---|---|---|
| WEED POLLENS | | | | |
| *Asterales* | | | | |
| Ambrosia | Amb a 1; antigen E | 38 | C | 8,20 |
| artemisiifolia | Amb a 2; antigen K | 38 | C | 8,21 |
| (short ragweed) | Amb a 3; Ra3 | 11 | C | 22 |
| | Amb a 5; Ra5 | 5 | C | 11,23 |
| | Amb a 6; Ra6 | 10 | C | 24,25 |
| | Amb a 7; Ra7 | 12 | P | 26 |
| | Amb a ? | 11 | C | 27 |
| Ambrosia trifida (giant ragweed) | Amb t 5; Ra5G | 4.4 | C | 9,10,28 |
| Artemisia vulgaris | Art v 1 | 27-29 | C | 28A |
| (mugwort) | Art v 2 | 35 | P | 29 |
| Helianthus annuus | Hel a 1 | 34 | - | 29a |
| (sunflower) | Hel a 2; profilin | 15.7 | C | Y15210 |
| Mercurialis annua | Mer a 1; profilin | 14-15 | C | Y13271 |
| GRASS POLLENS | | | | |
| Poales | | | | |
| Cynodon dactylon | Cyn d 1 | 32 | C | 30,S83343 |
| (Bermuda grass) | Cyn d 7 | | C | 31,X91256 |
| | Cyn d 12; profilin | 14 | C | 31a,Y08390 |
| Dactylis glomerata | Dac g 1; AgDg1 | 32 | P | 32 |
| (orchard grass) | Dac g 2 | 11 | C | 33,S45354 |
| | Dac g 3 | | C | 33a,U25343 |
| | Dac g 5 | 31 | P | 34 |
| Holcus lanatus (velvet grass) | Hol l 1 | | C | Z27084,Z68893 |
| Lolium perenne | Lol p 1; group I | 27 | C | 35,36 |
| (rye grass) | Lol p 2; group II | 11 | C | 37,37a,X73363 |
| | Lol p 3; group III | 11 | C | 38 |
| | Lol p 5 ; Lol p IX, | 31/35 | | 34,39 |
| | Lol p Ib | | | |
| | Lol p 11; trypsin | 16 | | 39a |
| | inh. Related | | | |
| Phalaris aquatica (canary grass) | Pha a 1 | | C | 40,S80654 |
| Phleum pratense | Phl p 1 | 27 | C | X78813 |
| (timothy grass) | Phl p 2 | | C | 41,X75925 |
| | Phl p 4 | | P | 41A |
| | Phl p 5; Ag25 | 32 | C | 42 |
| | Phl p 6 | | C | 43,Z27082 |
| | Phl p 12; profilin | | C | 44,X77583 |
| | Phl p 13; polygalacturonase | 55-60 | C | AJ238848 |
| Poa pratensis | Poa p 1; group I | 33 | P | 46 |
| (Kentucky blue grass) | Poa p 5 | 31/34 | C | 34,47 |
| Sorghum halepense (Johnson grass) | Sor h 1 | | C | 48 |
| TREE POLLENS | | | | |
| *Fagales* | | | | |
| Alnus glutinosa | Aln g 1 | 17 | C | S50892 |
| (alder) | | | | |
| Betula verrucosa | Bet v 1 | 17 | C | see list of isoallergens M65179 |
| | Bet v 2; profilin | 15 | C | X79267 |
| | Bet v 3 | 8 | C | X87153/SS4819 |
| | Bet v 4 | | C | AF135127 |
| | Bet v 5; isoflavone reductase | 33.5 | C | |
| | homologue | | | |
| | Bet v 7; cyclophilin | 18 | C | P P81531 |
| Carpinus betulus (hornbeam) | Car b 1 | 17 | C | 51 |
| Castanea sativa | Cas s 1; Bet v I homologue | 22 | P | 52 |
| (chestnut) | Cas s5; chitinase | | | |
| Corylus avelana (hazel) | Cor a 1 | 17 | C | 53 |
| Quercus alba (white oak) | Que a 1 | 17 | P | 54 |
| Cryptomeria japonica | Cry j 1 | 41-45 | C | 55,56 |
| (sugi) | Cry j 2 | | C | 57,D29772 |
| Juniperus ashei | Jun a 1 | 43 | P | P81294 |
| (mountain cedar) | Jun a 3 | 30 | P | P81295 |
| Juniperus oxycedrus | Jun o 2; calmodulin-like | 29 | C | AF031471 |
| (prickly juniper) | | | | |
| Juniperus sabinoides | Jun s 1 | 50 | P | 58 |
| (mountain cedar) | | | | |
| Juniperus virginiana | Jun v 1 | 43 | P | P81825 |
| (eastern red cedar) | | | | |
| *Oleales* | | | | |
| Fraxinus excelsior | Fra e 1 | 20 | P | 58A |
| (ash) | | | | |
| Ligustrum vulgare | Lig v 1 | 20 | P | 58A |
| (privet) | | | | |
| Olea europea | Ole e 1; | 16 | C | 59,60 |
| (olive) | Ole e 2; profilin | 15-18 | C | 60A |
| | Ole e 3; | 9.2 | | 60B |
| | Ole e 4; | 32 | P | P80741 |
| | Ole e 5; superoxide dismutase | 16 | P | P80740 |
| | Ole e 6; | 10 | C | U86342 |
| Syringa vulgaris | Syr v 1 | 20 | P | 58A |
| (lilac) | | | | |
| MITES | | | | |
| Acarus siro | Aca s 13; fatty acid-bind.prot. | 14* | C | AJ006774 |
| (mite) | | | | |
| Blomia tropicalis | Blo t 5; | | C | U59102 |
| (mite) | Blo t 12; Bt11a | | C | U27479 |
| | Blo t 13; Bt6 fatty acid-binding prot | | C | U58106 |
| Dermatophagoides pteronyssinus | Der p 1; antigen P1 | 25 | C | 61 |
| (mite) | Der p 2; | 14 | C | 62 |
| | Der p 3; trypsin | 28/30 | C | 63 |
| | Der p 4; amylase | 60 | C | 64 |
| | Der p 5; | 14 | P | 65 |
| | Der p 6; chymotrypsin | 25 | C | 66 |
| | Der p 7; | 22-28 | C | 67 |
| | Der p 8; glutathione transferase | | P | 67A |
| | Der p 9; collagenolytic serine prot. | | C | 67B |
| | Der p 10; tropomyosin | 36 | | Y14906 |
| | Der p 14; apolipophorin like p | | C | Epton p.c. |
| Dermatophagoides microceras (mite) | Der m 1; | 25 | P | 68 |
| Dermatophagoides farinae (mite) | Der f 1; | 25 | C | 69 |
| | Der f 2; | 14 | C | 70,71 |
| | Der f 3; | 30 | C | 63 |
| | Der f 10; tropomyosin | | C | 72 |
| | Der f 11; paramyosin | 98 | C | 72a |
| | Der f 14; Mag3, apolipophorin | | C | D17686 |
| Euroglyphus maynei | Eur m 14; apolipophorin | 177 | C | AF149827 |
| (mite) | | | | |
| Lepidoglyphus destructor | Lep d 2.0101; | 15 | C | 73,74,75 |
| (storage mite) | Lep d 2.0102; | 15 | C | 75 |
| ANIMALS | | | | |
| Bos domesticus | Bos d 2; Ag3,lipocalin | 20 | C | 76,L42867 |
| (domestic cattle) | Bos d 4; alpha-lactalbumin | 14.2 | C | M18780 |
| (see also foods) | Bos d 5; beta-lactoglobulin | 18.3 | C | X14712 |
| | Bos d 6; serum albumin | 67 | C | M73993 |
| | Bos d 7; immunoglobulin | 160 | | 77 |
| | Bos d 8; caseins | 20-30 | | 77 |
| Canis familiaris | Can f 1; | 25 | C | 78,79 |
| (Canis domestics | Can f 2; | 27 | C | 78,79 |
| (dog) | Can f ?; albumin | | C | S72946 |
| Equus caballus | Equ c 1; lipocalin | 25 | C | U70823 |
| (domestic horse) | Equ c 2; lipocali | 18.5 | P | 79A,79B |
| Felis domesticus | Fel d 1; cat-1 | 38 | C | 15 |
| (cat saliva) | | | | |
| Mus musculus | Mus m 1; MUP | 19 | C | 80,81 |
| (mouse urine) | | | | |
| Rattus norvegius | Rat n 1 | 17 | C | 82,83 |
| (rat urine) | | | | |
| FUNG1 | | | | |
| *Ascomycota* | | | | |
| Dothidiales | | | | |
| Altemaria alternata | Alt a 1; | 28 | C | U82633 |
| | Alt a 2; | 25 | C | U87807,U87808 |
| | Alt a 3; heat shock protein | 70 | C | X78222, |
| | Alt a 6; ribosomal protein | 11 | C | U87806 |
| | Alt a 7; YCP4 protein | 22 | C | X78225 |
| | Alt a 10; aldehyde dehydrogenase | 53 | C | X78127,P42041 |
| | Alt a 11; enolase | 45 | C | U82437 |
| | Alt a 12;acid.ribosomal prot P1 | 11 | C | X84216 |
| Cladosporium herbarum | Cla h 1; | 13 | | 83a,83b |
| | Cla h 2; | 23 | | 83a,83b |
| | Cla h 3; aldehyde dehydrogenase | 53 | C | X78228 |
| | Cla h 4; ribosomal protein | 11 | C | X78223 |
| | Cla h 5; YCP4 protein | 22 | C | X78224 |
| | Cla h 6; enolase | 46 | C | X78226 |
| | Cla h 12;acid.ribosomal prot P1 | 11 | C | X85180 |
| Eurotiales | | | | |
| | Asp fl 13; alkaline | 34 | | 84 |
| | serine proteinase | | | |
| Aspergillus Fumigatus | Asp f 1; | 18 | C | 83781,S39330 |
| | Asp f 2; | 37 | C | U56938 |
| | Asp f 3; peroxisomal protein | 19 | C | U20722 |
| | Asp f 4; | 30 | C | AJ001732 |
| | Asp f 5; metalloprotease | 42 | C | Z30424 |
| | Asp f 6; Mn superoxide dismutase | 26.5 | C | U53561 |
| | Asp f 7; | 12 | C | AJ223315 |
| | Asp f 8; ribosomal protein P2 | 11 | C | AJ224333 |
| | Asp f 9; | 34 | C | AJ223327 |
| | Asp f 10; aspartic protease | 34 | | X85092 |
| | Asp f 11; peptidyl-prolyl isom | 24 | | 84a |
| | Asp f 12; heat shock prot. P70 | 65 | C | U92465 |
| | Asp f 13; alkaline serine proteinase | 34 | | 84b |
| | Asp f 15; | 16 | C | AJ002026 |
| | Asp f 16; | 43 | C | g3643813 |
| | Asp f 17; | 34 | C | AJ224865 |
| | Asp f 18; vacuolar serine | 90 | | 84c |
| | Asp f ?; | 55 | P | 85 |
| | Asp f ?; | | P | 86 |
| Aspergillus niger | Asp n 14; beta-xylosidase | 105 | C | AF108944 |
| | Asp n 18; | 34 | C | 84b |
| | vacuolar serine | | | |
| | proteinase | | | |
| | Asp n ?; | 85 | C | Z84377 |
| Aspergillus oryzae | Asp o 2; TAKA amylase A | 53 | C | D00434,M33218 |
| | Asp o 13; alkaline serine proteinase | 34 | C | X17561 |
| Penicillium brevicompactum | Pen b 13; alkaline serine Proteinase | 33 | | 86a |
| Penicillium citrinum | Pen c 1; heat shock protein P70 | 70 | C | U64207 |
| | Pen c 3; peroxisomal membrane protein | | | 86b |
| | Pen c 13; alkaline serine proteinase | 33 | | 86a |
| Penicillium notatum | Pen n 1; N-acetyl glucosaminidase | 68 | | 87 |
| | Pen n 13; alkaline serine proteinase | 34 | | 89 |
| | Pen n 18; vacuolar serine proteinase | 32 | | 89 |
| Penicillium oxalicum Onygenales | Pen o 18; vacuolar serine proteinase | 34 | | 89 |
| Onygenales | | | | |
| Trichophyton rubrum | Tri r 2; | | C | 90 |
| | Tri r 4; serine protease | | C | 90 |
| Trichophyton tonsurans | Tri t 1; | 30 | P | 91 |
| | Tri t 4; serine protease | 83 | C | 90 |
| Saccharomycetales | | | | |
| Candida albicans | Cand a 1 | 40 | C | 88 |
| Candida boidinii | Cand b 2 | 20 | C | J04984,J04985 |
| *Basidiomycota* | | | | |
| Basidiolelastomycetes | | | | |
| Malassezia furfur | Mal f 1; | | | 91a |
| | Mal f 2; MF1 peroxisomal | 21 | C | AB011804 |
| | membrane protein | | | |
| | Mal f 3; MF2 peroxisomal | 20 | C | AB011805 |
| | membrane protein | | | |
| | Mal f 4, | 35 | C | Takesako, p.c. |
| | Mal f 5; | 18* | C | AJ011955 |
| | Mal f 6; cyclophilin homologue | 17* | C | AJ011956 |
| Basidiomycetes | | | | |
| Psilocybe cubensis | Psi c 1; | 16 | | 91b |
| | Psi c 2; cyclophilin | | | |
| Coprinus comatus | Cop c 1; | 11 | C | AJ132235 |
| (shaggy cap) | Cop c 2; | | | |
| | Cop c 3; | | | Brander, p.c. |
| | Cop c 5; | | | Brander, p.c. |
| | Cop c 7; | | | Brander, p.c. |
| INSECTS | | | | |
| Aedes aegyptii | Aed a 1; apyrase | 68 | C | L12389 |
| (mosquito) | Aed a 2; | 37 | C | M33157 |
| Apis mellifera | Api m 1; phospholipase A2 | 16 | C | 92 |
| (honey bee) | Api m 2; hyaluronidase | 44 | C | 93 |
| | Api m 4; melittin | 3 | C | 94 |
| | Api m 6; | 7-8 | P | Kettner,p.c. |
| Bombus pennsylvanicus (bumble bee) | Bom p 1; phospholipase | 16 | P | 95 |
| | Bom p 4; protease | | P | 95 |
| Blattella germanica | Bla g 1; Bd90k | | C | 96 |
| (German cockroach) | Bla g 2; aspartic protease | 36 | C | |
| | Bla g 4; calycin | 21 | C | 97 |
| | Bla g 5; glutathione transf. | 22 | C | 98 |
| | Bla g 6; troponin C | 27 | C | 98 |
| Periplaneta americana | Per a 1; Cr-PII | 72-78 | C | 98A |
| (American cockroach) | Per a 3; Cr-PI | | C | |
| | Per a 7; tropomyosin | 37 | C | Y14854 |
| Chironomus thummi | Chi t 1-9; hemoglobin | 16 | C | 99 |
| thummi (midges) | Chi t 1.01; component III | 16 | C | P02229 |
| | Chi t 1.02; component IV | 16 | C | P02230 |
| | Chi t 2.0101; component I | 16 | C | P02221 |
| | Chi t 2.0102; component IA | 16 | C | P02221 |
| | Chi t 3; component II-beta | 16 | C | P02222 |
| | Chi t 4; component IIIA | 16 | C | P02231 |
| | Chi t 5; component VI | 16 | C | P02224 |
| | Chi t 6.01; component VIIA | 16 | C | P02226 |
| | Chi t 6.02; component IX | 16 | C | P02223 |
| | Chi t 7; component VIIB | 16 | C | P02225 |
| | Chi t 8; component VIII | 16 | C | P02227 |
| | Chi t 9; component X | 16 | C | P02228 |
| Dolichovespula | Dol m 1; phospholipase A I | 35 | C | 100 |
| maculata | Dol m 2; hyaluronidase | 44 | C | 101 |
| (white face hornet) | Dol m 5; antigen 5 | 23 | C | 102,103 |
| Dolichovespula | Dol a 5; antigen 5 | 23 | C | 104 |
| arenaria | | | | |
| (yellow hornet) | | | | |
| Polistes annularies | Pol a 1; phospholipase A1 | 35 | P | 105 |
| (wasp) | Pol a 2; hyaluronidase Pol a 5; antigen 5 | 44 | P | 105 |
| | | 23 | C | 104 |
| Polistes dominulus | Pol d 1; | 32-34 | C | DR Hoffman |
| (Mediterranean paper | Pol d 4; serine protease | | | DR Hoffman |
| wasp) | Pol d 5; | | | P81656 |
| Polistes exclamans | Pol e 1; phospholipase A1 | 34 | P | 107 |
| (wasp) | Pol e 5; antigen 5 | 23 | C | 104 |
| Polistes fuscatus (wasp) | Pol f 5; antigen 5 | 23 | C | 106 |
| Polistes metricus | Pol m 5; antigen 5 | 23 | P | 106 |
| (wasp) | | | | |
| Vespa crabo | Vesp c 1; phospholipase | 34 | P | 107 |
| (European hornet) | Vesp c 5.0101; antigen 5 | 23 | C | 106 |
| | Vesp c 5.0102; antigen 5 | 23 | C | 106 |
| Vespa mandarina | Vesp m 1.01; | | | DR Hoffman |
| (giant asian hornet) | Vesp m 1.02; | | | DR Hoffman |
| | Vesp m 5; | | | P81657 |
| Vespula flavopilosa | Ves f 5; antigen 5 | 23 | C | 106 |
| (yellowjacket) | | | | |
| Vespula germanica | Ves g 5; antigen 5 | 23 | C | 106 |
| (yellowjacket) | | | | |
| Vespula maculifrons | Ves m 1; phospholipase A1 | 33.5 | C | 108 |
| (yellowjacket) | Ves m 2; hyaluronidase | 44 | P | 109 |
| | Ves m 5; antigen 5 | 23 | 23 | 104 |
| Vespula | Ves p 5; antigen 5 | 23 | C | 106 |
| pennsylvanica | | | | |
| (yellowjacket) | | | | |
| Vespula squamosa (yellowjacket) | Ves s 5; antigen 5 | 23 | C | 106 |
| Vespula vidua (wasp) | Ves vi 5; | 23 | C | 106 |
| Vespula vulgaris | Ves v 1; phopholipase A1 | 35 | C | 105A |
| (yellowjacket) | Ves v 2; hyaluronidase | 44 | P | 105A |
| | Ves v 5; antigen 5 | 23 | C | 104 |
| Myrmecia pilosula (Australian jumper ant) | Myr p 1, | | C | X70256 |
| | Myr p 2; | | C | S81785 |
| Solenopsis geminata | Sol g 2; | | | DR Hoffman |
| (tropical fire ant) | Sol g 4 | | | DR Hoffman |
| Solenopsis invicta | Sol i 2; | 13 | C | 110,111 |
| (fire ant) | Sol i 3; | 24 | C | 110 |
| | Soli 4; | 13 | C | 110 |
| Solenopsis saevissima | Sols 2; | | | DR Hoffman |
| (brazilian fire ant) | | | | |
| FOODS | | | | |
| Gadus callarias | Gad c 1; allergen M | 12 | C | 112,113 |
| (cod) | | | | |
| Salmo salar | Sals 1; parvalbumin | 12 | C | X97824,X97825 |
| (Atlantic salmon) | | | | |
| Bos domesticus | Bos d 4; alpha-lactalbumin | 14.2 | C | M18780 |
| (domestic cattle) | Bos d 5; beta-lactoglobulin | 18.3 | C | X14712 |
| | Bos d 6; serum albumin | 67 | C | M73993 |
| | Bos d 7; immunoglobulin | 160 | | 77 |
| | Bos d 8; caseins | 20-30 | | 77 |
| Gallus domesticus | Gal d 1; ovomucoid | 28 | C | 114,115 |
| (chicken) | Gald 2; ovalbumin | 44 | C | 114,115 |
| | Gald 3; conalbumin (Ag22) | 78 | C | 114,115 |
| | Gald 4; lysozyme | 14 | C | 114,115 |
| | Gal d 5; serum albumin | 69 | C | X60688 |
| Metapenaeus ensis (shrimp) | Met e 1; tropomyosin | | C | U08008 |
| Penaeus aztecus (shrimp) | Pen a 1; tropomyosin | 36 | P | 116 |
| Penaeus indicus (shrimp) | Pen i 1; tropomyosin | 34 | C | 117 |
| Todarodes pacificus (squid) | Tod p 1; tropomyosin | 38 | P | 117A |
| Haliotis Midae (abalone) | Hal m 1 | 49 | | 117B |
| Apium graveolens | Api g 1; Bet v 1 homologue | 16* | C | Z48967 |
| (celery) | Api g 4; profilin | | | AF129423 |
| | Api g 5; | 55/58 | P | P81943 |
| Brassica juncea | Bra j 1; 2S albumin | 14 | C | 118 |
| (oriental mustard) | | | | |
| Brassica rapa | Bra r 2; prohevein-like protein | 25 | ? | P81729 |
| (turnip) | | | | |
| Hordeum vulgare | Hor v 1; BMAI-1 | 15 | C | 119 |
| (barley) | | | | |
| Zea mays | Zea m 14; lipid transfer prot. | 9 | P | P19656 |
| (maize, corn) | | | | |
| Corylus avellana | Cor a 1.0401; Bet v 1 | 17 | C | AF136945 |
| (hazelnut) | homologue | | | |
| Malus domestica | Mal d 1; Bet v 1 homologue | | C | X83672 |
| (apple) | Mal d 3; lipid transfer protein | 9 | C | Pastorello |
| Pyrus communis | Pyr c 1; Bet v 1 homologue | 18 | C | AT05730 |
| (pear) | Pyr c 4; profilin | 14 | C | AF129424 |
| | Pyr c 5; isoflavone reductase | | | |
| | homologue | 33.5 | C | AF071477 |
| Oryza sativa (rice) | Ory s 1; | | C | U31771 |
| Persea americana (avocado) | Pers a 1; endochitinase | 32 | C | Z78202 |
| Prunus armeniaca | Pru ar 1; Bet v 1 homologue | | C | U93165 |
| (apricot) | Pru ar 3; lipid transfer protein | 9 | P | |
| Prunus avium | Pru av 1; Bet v 1 homologue | | C | U66076 |
| (sweet cherry) | Pru av 2; thaumatin homologue | | C | U32440 |
| | Pru av 4; profilin | 15 | C | AF129425 |
| Prunus persica (peach) | Pru p 3;lipid transfer protein | 10 | P | P81402 |
| Sinapis alba (yellow mustard) | Sin a 1; 2S albumin | 14 | C | 120 |
| Glycine max | Gly m 1.0101; HPS | 7.5 | P | 121 |
| (soybean) | Gly m 1.0102; HPS | 7 | P | 121 |
| | Gly m 2 | 8 | P | A57106 |
| | Gly m 3; profilin | 14 | C | AJ223982 |
| Arachis hypogaea | Ara h 1; vicilin | 63.5 | C | L34402 |
| (peanut) | Ara h 2; conglutin | 17 | C | L77197 |
| | Ara h 3; glycinin | 14 | C | AF093541 |
| | Ara h 4; glycinin | 37 | C | AF086821 |
| | Ara h 5; profilin | 15 | C | AF059616 |
| | Ara h 6; conglutin homolog | 15 | C | AF092846 |
| | Ara h 7; conglutin homolog | 15 | C | AF091737 |
| Actinidia chinensis (kiwi) | Act c 1; cysteine protease | 30 | P | P00785 |
| Solanum tuberosum (potato) | Sol t 1; patatin | 43 | P | P15476 |
| Bertholletia excelsa (Brazil nut) | Ber e 1; 2S albumin | 9 | C | P04403,M17146 |
| Juglans regia | Jug r 1; 2S albumin | 44 | C | U66866 |
| (English walnut) | Jug r 2; vicilin | | C | AF066055 |
| Ricinus communis | Ric c 1; 2S albumin | | C | P01089 |
| (Castor bean) | | | | |
| OTHERS | | | | |
| Anisakis simplex | Ani s 1 | 24 | P | A59069 |
| (nematode) | Ani s 2; paramyosin | 97 | C | AF173004 |
| Ascaris suum | Asc s 1; | 10 | P | 122 |
| (worm) | | | | |
| Aedes aegyptii | Aed a 1; apyrase | 68 | C | L12389 |
| (mosquito) | Aed a 2; | 37 | C | M33157 |
| Hevea brasiliensis | Hev b 1; elongation factor | 58 | P | 123,124 |
| (rubber) | Hev b 2; ( 1,3-glucanase | 58 | P | 123,124 |
| | Hev b 2; ( 1,3-glucanase | 34/36 | C | 125 |
| | Hev b 3 | 24 | P | 126,127 |
| | Hev b 4; component of | 100/110/115 | P | 128 |
| | microhelix protein complex | | | |
| | Hev b 5 | 16 | C | U42640 |
| | Hev b 6.01 hevein precursor | 20 | C | M36986/p02877 |
| | Hev b 6.02 hevein | 5 | C | M36986/p02877 |
| | Hev b 6.03 C-terminal fragment | 14 | C | M36986/p02877 U80598 |
| | Hev b 7; patatin homologue | 46 | C | Y15042 |
| | Hev b 8; profilin | 14 | C | AJ132580/AJ1 |
| | Hev b 9; enolase | 51 | C | 32581 |
| | | 26 | | AJ249148 |
| | Hev b 10; Mn-superoxide | | C | |
| | dismut | | | |
| Ctenocephalides felis | Cte f 1; | | | |
| felis (cat flea) | Cte f 2; Mlb | 27 | C | AF231352 |
| Homo sapiens | Hom s 1; | 73* | C | Y14314 |
| (human | Hom s 2; | 10.3* | C | X80909 |
| autoallergens) | Hom s 3; | 20.1* | C | X89985 |
| | Hom s 4; | 36* | C | Y17711 |
| | Hom s 5; | 42.6* | C | P02538 |

1. Marsh, D.G., and L.R. Freidhoff. 1992. ALBE, an allergen database. IUIS, Baltimore, MD, Edition 1.0.
2. Marsh, D. G., L. Goodfriend, T. P. King, H. Lowenstein, and T. A. E. Platts-Mills. 1986. Allergen nomenclature. Bull WHO 64:767-770.
3. King. T.P., P.S. Norman, and J.T. Cornell. 1964. Isolation and characterization of allergen from ragweed pollen. II. Biochemistry 3:458-468.
4. Lowenstein, H. 1980. Timothy pollen allergens. Allergy 35:188-191.
5. Aukrust, L. 1980. Purification of allergens in Cladosporium herbarum. Allergy 35:206-207.
6. Demeree, M., E. A. Adelberg, A. J. Clark, and P. E. Hartman. 1966. A proposal for a uniform nomenclature in bacterial genetics. Genetics 54:61-75.
7. Bodmer, J. G., E. D. Albert, W. F. Bodmer, B. Dupont, H. A. Erlich, B. Mach. S. G. E. Marsh, W. R. Mayr, P. Parham, T. Sasuki, G. M. Th. Schreuder, J. L. Strominger, A. Svejgaard, and P. 1. Terasaki. 1991. Nomenclature for factors of the HLA system, 1990. Immunogenetics 33:301-309.
8. Griffith, I.J., J. Pollock, D.G. Klapper, B.L. Rogers, and A.K. Nault. 1991. Sequence polymorphism of Amb a 1 and Amb a II, the major allergens in Ambrosia artemisiifolia (short ragweed). Int. Arch. Allergy Appl. Immunol. 96:296-304.
9. Roebber, M., D. G. Klapper, L. Goodfriend, W. B. Bias, S. H. Hsu, and D. G. Marsh. 1985. Immunochemical and genetic studies of Amb t V (Ra5G), an Ra5 homologue from giant ragweed pollen. J. Immunol. 134:3062-3069.
10. Metzler, W. J., K. Valentine, M. Roebber, M. Friedrichs, D. G. Marsh, and L. Mueller. 1992. Solution structures of ragweed allergen Amb t V. Biochemistry 31:5117-5127.
11. Metzler, W. J., K. Valentine, M. Roebber, D. G. Marsh, and L. Mueller. 1992. Proton resonance assignments and three-dimensional solution structure of the ragweed allergen Amb a V by nuclear magnetic resonance spectroscopy. Biochemistry 31:8697-8705.
12. Goodfriend, L., A.M. Choudhury, J. Del Carpio, and T.P. King. 1979. Cytochromes C: New ragweed pollen allergens. Fed. Proc. 38:1415.
13. Ekramoddoullah, A. K. M., F. T. Kisil, and A. H. Sehon. 1982. Allergenic cross reactivity of cytochrome c from Kentucky bluegrass and perennial ryegrass pollens. Mol. Immunol. 19:1527-1534.
14. Ansari, A. A., E. A. Killoran, and D. G. Marsh. 1987. An-investigation of human response to perennial ryegrass (Lolium perenne) pollen cytochrome c (Lol p X). J. Allergy Clin. Immunol. 80:229-235.
15. Morgenstem, J.P., I.J. Griffith, A.W. Brauer, B.L. Rogers, J.F. Bond, M.D. Chapman, and M. Kuo. 1991. Amino acid sequence of Fel d 1, the major allergen of the domestic cat: protein sequence analysis and cDNA cloning. Proc. Natl. Acad. Sci. USA 88:9690-9694.
16. Griffith, I.J., S. Craig, J. Pollock, X. Yu, J.P. Morgenstern, and B.L.Rogers. 1992. Expression and genomic structure of the genes encoding Fdl, the major allergen from the domestic cat. Gene 113:263-268.
17. Weber, A., L. Marz, and F. Almann. 1986. Characteristics of the asparagine-linked oligosaccharide from honey-bee venom phospholipase A2. Comp. Biochem. Physiol. 83B:321-324.
18. Weber, A., H. Schroder, K. Thalberg, and L. Marz. 1987. Specific interaction of IgE antibodies with a carbohydrate epitope of honey bee venom phospholipase A2. Allergy 42:464-470.
19. Stanworth, D. R, K. J. Dorrington, T. E. Hugli, K. Reid, and M. W. Turner. 1990. Nomenclature for synthetic peptides representative of immunoglobulin chain sequences. Bulletin WHO 68:109-111.
20. Rafnar, T., I. J. Griffith, M. C. Kuo, J. F. Bond, B. L. Rogers, and D.G. Klapper. 1991. Cloning of Amb a I (Antigen E), the major allergen family of short ragweed pollen. J. Biol. Chem. 266: 1229-1236.
21. Rogers, B.L., J.P. Morgenstem, I.J. Griffith, X.B. Yu, C.M. Counsell, A.W. Brauer, T.P. King, R.D. Garman, and M.C. Kuo. 1991. Complete sequence of the allergen Amb a II: recombinant expression and reactivity with T cells from ragweed allergic patients. J. Immunol. 147:2547-2552.
22. Klapper, D.G., L. Goodfriend, and J.D. Capra. 1980. Amino acid sequence of ragweed allergen Ra3. Biochemistry 19:5729-5734.
23. Ghosh, B., M.P. Perry, T. Rafnar, and D.G. Marsh. 1993. Cloning and expression of immunologically active recombinant Amb a V allergen of short ragweed (Ambrosia artemisiifolia) pollen. J. Immunol. 150:5391-5399.
24. Roebber, M., R. Hussain, D. G. Klapper, and D. G. Marsh. 1983. Isolation and properties of a new short ragweed pollen allergen, Ra6. J. Immunol. 131:706-711.
25. Lubahn, B., and D.G. Klapper. 1993. Cloning and characterization of ragweed allergen Amb a VI (abst). J. Allergy Clin. Immunol. 91:338.
26. Roebber, M., and D.G. Marsh. 1991. Isolation and characterization of allergen Amb a VII from short ragweed pollen. J. Allergy Clin. Immunol. 87:324.
27. Rogers, B.I., J. Pollock. D.G. Klapper, and I.J. Griffith. 1993. Cloning, complete sequence, and recombinant expression of a novel allergen from short ragweed pollen (abst). J. Allergy Clin. Immunol. 91:339.
28. Goodfriend, L., A.M. Choudhury, D.G. Klapper, K.M. Coulter, G. Dorval, J. DelCarpio, and C.K. Osterland. 1985. Ra5G, a homologue of Ra5 in giant ragweed pollen: isolation, HLA-DR-associated activity and amino acid sequence. Mol. Immunol. 22:899-906.
28A. Breitenbach M, pers. comm.
29. Nilsen, B. M., K. Sletten, M. O'Neill, B. Smestead Paulsen, and H. van Halbeek. 1991. Structural analysis of the glycoprotein allergen Art v II from pollen of mugwort (Anenesia vulgaris). J. Biol. Chem. 266:2660-2668.
29A Jimenez A, Moreno C, Martinez J, Martinez A, Bartolome B, Guerra F, Palacios R 1994. Sensitization to sunflower pollen: only an occupational allergy? Int Arch Allergy Immunol 105:297-307.
30. Smith,P.M., Suphioglu,C., Griffith,I.J., Theriault,K., Knox.R.B. and Singh,M.B. 1996. Cloning and expression in yeast Pichia pastoris of a biologically active form of Cyn d 1, the major allergen of Bermuda grass pollen. J. Allergy Clin. Immunol. 98:331-343.
31. Suphioglu,C., Ferreira,F. and Knox,R.B. 1997. Molecular cloning and immunological characterisation of Cyn d 7, a novel calcium-binding allergen from Bermuda grass pollen. FEBS Lett. 402:167-172.
31a. Asturias JA, Arilla MC, Gomez-Bayon N, Martinez J, Martinez A, and Palacios R. 1997. Cloning and high level expression of Cynodon dactylon (Bermuda grass) pollen profilin (Cyn d 12) in Escherichia coli: purification and characterization of the allergen. Clin Exp Allergy 27:1307-1313.
32. Mecheri, S., G. Peltre, and B. David. 1985. Purification and characterization of a major allergen from Dactylis glomerata pollen: The Ag Dg I. Int. Arch. Allergy Appl. Immunol. 78:283-289.
33. Roberts, A.M., LJ. Bevan, P.S. Flora, I. Jepson, and M.R. Walker. 1993. Nucleotide sequence of cDNA encoding the Group II allergen of Cocksfoot/Orchard grass (Dactylis glomerata), Dac g II. Allergy 48:615-623.
33a. Guerin-Marchand,C., Senechal,H., Bouin,A.P., Leduc-Brodard,V., Taudou,G., Weyer,A., Peltre,G. and David.B. 1996. Cloning, sequencing and immunological characterization of Dac g 3, a major allergen from Dactylis is glomerata pollen. Mol. Immunol. 33:797-806.
34. Klysner, S., K. Welinder, H. Lowenstein, and F. Matthiesen. 1992. Group V allergens in grass pollen IV. Similarities in amino acid compositions and amino terminal sequences of the group v allergens from Lolium perenne, Poa pratensis and Dactylis glomerata. Clin. Exp. Allergy 22: 491-497.
35. Perez, M., G. Y. Ishioka, L. E. Walker, and R. W. Chesnut. 1990. cDNA cloning and immunological characterization of the rye grass allergen Loi p I. J. Biol. Chem. 265:16210-16215.
36. Grifith, I. J., P. M. Smith, J. Pollock, P. Theerakulpisut, A. Avjioglu, S. Davies, T. Hough, M. B. Singh, R. J. Simpson, L. D. Ward, and R. B. Knox. 1991. Cloning and sequencing of Lol p 1, the major allergenic protein of rye-grass pollen. FEBS Letters 279:210-215.
37. Ansari, A. A., P. Shenbagamurthi, and D.G. Marsh. 1989. Complete amino acid sequence of a Lolium perenne (perennial rye grass) pollen allergen, Lol p II. J. Biol. Chem. 264:11181-11185.
37a. Sidoli,A., Tamborini,E., Giuntini,J., Levi,S., Volonte,G., Paini,C., De Lalla,C., Siccardi,A.G., Baralie,F.E., Galliani,S. and Arosio,P. 1993. Cloning, expression, and immunological characterization of recombinant Lolium perenne allergen Lol p II. J. Biol. Chem. 268:21819-21825.
38. Ansari, A. A., P. Shenbagamurthi, and D. G. Marsh. 1989. Complete primary structure of a Lolium perenne (perennial rye grass) pollen allergen, Lol p III: Comparison with known Lol p 1 and II sequences. Biochemistry 28:8665-8670.
39. Singh, M. B., T. Hough, P. Theerakulpisut, A. Avjioglu, S. Davies, P. M. Smith, P. Taylor, R. J. Simpson, L. D. Ward, J. McCluskey, R. Puy, and R.B. Knox. 1991. Isolation of cDNA encoding a newly identified major allergenic protein of rye-grass pollen: Intracellular targeting to the amyloplost. Proc. Natl. Acad. Sci. 88:1384-1388.
39a. van Ree R, Hoffman DR, van Dijk W, Brodard V, Mahieu K, Koeleman CA, Grande M, van Leeuwen WA, Aalberse RC. 1995. Lol p XI, a new major grass pollen allergen, is a member of a family of soybean trypsin inhibitor-related proteins. J Allergy Clin Immunol 95:970-978.
40. Suphioglu,C. and Singh,M.B. 1995. Cloning, sequencing and expression in Escherichia coli of Pha a I and four isoforms of Pha a 5, the major allergens of canary grass pollen. Clin. Exp. Allergy 25:853-865.
41. Dolecek,C., Vrtala,S., Laffer,S., Steinberger,P., Kraft,D., Scheiner,O. and Valenta,R. 1993. Molecular characterization of Phl p II, a major timothy grass (Phleum pretense) pollen allergen. FEBS Lett. 335:299-304.
41A. Fischer S, Grote M, Fahlbusch B, Muller WD, Kraft D, Valenta R. 1996. Characterization of Phl p 4, a major timothy grass (Phleum pratense) pollen allergen. J Allergy Clin Immunol 98:189-198.
42. Matthiesen, F., and H. Lowenstein. 1991. Group V allergens in grass pollens. L Purification and characterization of the group V allergen from Phleum pratense pollen, Phl p V. Clin. Exp. Allergy 21:297-307.
43. Petersen,A., Bufe,A., Schramm,G., Schlaak,M. and Becker,W.M. 1995. Characterization of the allergen group VI in timothy grass pollen (Phl p 6). II. cDNA cloning of Phl p 6 and structural comparison to grass group v. Int. Arch. Allergy Immunol. 108:55-59.
44. Valenta,R., Ball,T., Vrtala,S., Duchene.M., Kraft,D. and Scheiner,O. 1994. cDNA cloning and expression of timothy grass (Phleum pratense) pollen profilin in Escherichia coli: comparison with birch pollen profilin. Biochem. Biophys. Res. Commun. 199:106-118.
46. Esch, R. E., and D. G. Klapper. 1989. Isolation and characterization of a major cross-reactive grass group 1 allergenic determinant. Mol. Immunol. 26:557-561.
47. Olsen, E., L. Zhang, R. D. Hill, F. T. Kisil, A. H. Schon, and S. Mohapatra. 1991. Identification and characterization of the Poa p IX group of basic allergens of Kentucky bluegrass pollen. J. Immunol. 147:205-211.
48. Avjioglu, A., M. Singh, and R.B. Knox. 1993. Sequence analysis of Sor h 1, the group I allergen of Johnson grass pollen and it comparison to rye-grass Lot p I (abst). J. Allergy Clin. Immunol. 91:340.
51. Larsen, J.N., P. Str-man, and H. Ipsen. 1992. PCR based cloning and sequencing of isogenes encoding the tree pollen major allergen Car b 1 from Carpinus betulus, hornbeam. Mol. Immunol. 29:703-711.
52. Kos T, Hoffmann-Sommergruber K, Ferreira F, Hirschwehr R, Ahom H, Horak F, Jager S, Sperr W, Kraft D, Scheiner O. 1993. Purification, characterization and N-terminal amino acid sequence of a new major allergen from European chestnut pollen-Cas s 1. Biochem Biophys Res Commun 196:1086-92.
53. Breiteneder, H., F. Ferreira, K. Hoffman-Sommergruber, C. Ebner, M. Breitenbach, H. Rumpoid, D. Kraft, and O. Scheiner. 1993. Four recombinant isoforms of Cor a 1, the major allergen of hazel pollen. Europ. J. Biochem. 212:355-362.
54. Ipsen, H., and B.C. Hansen. 1991. The NH2-terminal amino acid sequence of the immunochemically partial identical major allergens of alder (Alnus glutinosa) Aln g 1, birch (Betula verrucosa) Bet v 1, hornbeam (Carpinus betulus) Car b I and oak (Quercus alba) Que a I pollens. Mol. Immunol. 28:1279-1288.
55. Taniai, M., S. Ando, M. Usui, M. Kurimoto, M. Sakaguchi, S. Inouye, and T. Matuhasi. 1988. N-terminal amino acid sequence of a major allergen of Japanese cedar pollen (Cry j 1). FEBS Lett. 239:329-332.
56. Griffith, I.J., A. Lussier, R. Garman, R. Koury, H. Yeung, and J. Pollock. 1993. The cDNA cloning of Cry j 1, the major allergen of Cryptomeria japonica (Japanese cedar) (abst). J. Allergy Clin. Immunol. 91:339.
57. Sakaguchi, M., S. Inouye, M. Taniai, S. Ando, M. Usui, and T. Matuhasi. 1990. Identification of the second major allergen of Japanese cedar pollen. Allergy 45:309-312. 58 Gross GN, Zimburean JM, Capra JD 1978. Isolation and partial characterization of the allergen in mountain cedar pollen. Scand J Immunol 8:437-41
58A Obispo TM, Melero JA, Carpizo JA, Carreira J, Lombardero M 1993. The main allergen of Olea europaea (Ole e 1) is also present in other species of the oleaceae family. Clin Exp Ailergy 23:311-316.
59. Cardaba, B., D. Hernandez, E. Martin, B. de Andres, V. del Pozo, S. Gallardo, J.C. Fernandez, R. Rodriguez, M. Villalba, P. Palomino, A. Basomba, and C. Lahoz. 1993. Antibody response to olive pollen antigens: association between HLA class II genes and IgE response to Ole e I (abst). J. Allergy Clin. Immunol. 91:338.
60. Villalba, M., E. Batanero, C. Lopez-Otin, L.M. Sanchez, R.I. Monsalve, M.A. Gonzalez de la Pena, C. Lahoz, and R. Rodriguez. 1993. Amino acid sequence of Ole e 1, the major allergen from olive tree pollen (Olea curopaca). Europ.J. Biochem. 216:863-869.
60A. Asturias JA, Arilla MC, Gomez-Bayon N, Martinez J, Martinez A, Palacios R 1997. Cloning and expression of the panallergen profilin and the major allergen (Ole c 1) from olive tree pollen. J Allergy Clin Immunol 100:365-372.
60B. Batanero E, Villalba M, Ledesma A Puente XS, Rodriguez R. 1996. Ole e 3, an olive-tree allergen, belongs to a widesprcad family of pollen proteins. Eur J Biochem 241: 772-778.
61. Chua, K. Y., G. A. Stewart, and W. R. Thomas. 1988. Sequence analysisol cDNA encoding for a major house dust mite allergen, Der p I. J. Exp. Med. 167:175-182.
62. Chua, K. Y., C. R. Doyle, R. J. Simpson, K. J. Turner, G. A. Stewart, and W. R. Thomas. 1990. Isolation of CDNA coding for the major mite allergen Der p II by IgE plaque immunoassay. Int. Arch. Allergy Appl. Immunol. 91:118-123.
63. Smith WA, Thomas WR. 1996. Comparative analysis of the genes encoding group 3 allergens from Dermatophagoides pteronyssinus and Dermatophagoides farinae. Int Arch Allergy Immunol 109: 133-40.
64. Lake, F.R., L.D. Ward, R.J. Simpson, PJ. Thompson, and G.A. Stewart. 1991. House dust mite-derived amylase: Allergenicity and physicochemical characterisation. J. Allergy Clin. Immunol. 87:1035-1042.
65. Tovey, E. R., M. C. Johnson, A. L. Roche, G. S. Cobon, and B. A. Baldo. 1989. Cloning and sequencing of a cDNA expressing a recombinant house dust mite protein that binds human IgE and corresponds to an important low molecular weight allergen. J. Exp. Med. 170:1457-1462.
66. Yasueda, H., T. Shida, T. Ando, S. Sugiyama, and H. Yamakawa. 1991. Allergenic and proteolytic properties of fourth allergens from Dermatophagoides mites. In: "Dust Mite Allergens and Asthma. Report of the 2nd international workshop" A. Todt, Ed., UCB Institute of Allergy, Brussels, Belgium, pp. 63-64.
67. Shen, H.-D., Y.-Y. Chua, K.-L. Lin, K.-H. Hsich, and W.R. Thomas. 1993. Molecular cloning of a house dust mite allergen with common antibody binding specificities with multiple components in mite extracts. Clin. Exp. Allergy 23:934-40.
67A. O'Neil GM, Donovan GR, Baldo BA. 1994. Cloning and charaterisation of a major allergen of the house dust mite Dermatophagoides pteronyssinus, homologous with glutathione S-transferase. Biochim Biophys Acta, 1219:521-528.
67B. King C, Simpson RJ, Moritz RI., Reed GE, Thompson PJ, Stewart GA. 1996. The isolation and characterization of a novel collagenolytic serine protease allergen (Der p 9) from the dust mite Dermatophagoides pteronyssinus. J Allergy Clin Immunol 98:739-47.
68. Lind P, Hansen OC, Hom N. 1988. The binding of mouse hybridoma and human IgE antibodies to the major fecal allergen, Der p 1 of D. pteronyssinus. J. Immunol. 140:42564262.
69. Dilworth, R. J., K. Y. Chua, and W. R. Thomas. 1991. Sequence analysis ofcDNA coding for a mojor house dust allergn Der f1. Clin. Exp. Allergy 21:25-32.
70. Nishiyama, C., T. Yunki, T. Takai, Y. Okumura, and H. Okudaira. 1993. Determination of three disulfide bonds in a major house dust mite allergen, Der f II. Int. Arch. Allergy Immunol. 101:159-166.
71. Trudinger, M., K. Y. Chua, and W. R. Thomas. 1991. cDNA encoding the major dust mite allergen Der f II. Clin. Exp. Allergy 21:33-38.
72. Aki T, Kodama T, Fujikawa A, Miura K, Shigeta S, Wada T, Jyo T, Murooka Y, Oka S, Ono K. 1995. Immunochemical characteristion of recombinant and native tropomyosins as a new allergen from the house dust mite Dermatophagoides farinae, J Allergy Clin Immunol 96:74-83.
73. van Hage-Hamsten M., T. Bergman, E. Johansson, B. Persson, H. Jomvall, B. Harfast, and S.G.O. Johansson. 1993. N-terminal amino acid sequence of major allergen of the mite lepidoglyphus destructor (abst). J. Allergy Clin. Immunol. 91:353.
74. Varela J, Ventas P, Carreira J, Barbas JA, Gimenez-Gallego G, Polo F. Primary structure of Lep d I, the main Lepidoglyphus destructor allergen. Eur J Biochem 225:93-98, 1994.
75. Schmidt M, van der Ploeg I, Olsson S, van Hage Hamsten M. The complete cDNA encoding the Lepidoglyphus destructor major allergen Lep d I. FEBS Lett 370:11-14, 1995.
76. Rautiainen J, Rytkonen M, Pelkonen J, Pentikainen J, Perola O, Virtanen T, Zeiler T, Mantyjarvi R. BDA20, a major bovine dander allergen characterized at the sequence level is Bos d 2. Submitted.
77. Gjesing B, Lowenstein H. Immunochemistry of food antigens. Ann Allergy 53:602, 1984.
78. de Croot, H., K.G.H. Goei, P. van Swieten, and R.C. Aalberse. 1991. Affinity purification of a major and a minor allergen from dog extract: Serologic activity of affity-purified Can f I and Can f I-depleted extract. J. Allergy Clin. Immunol. 87:1056-1065.
79. Konieczny, A. Personal communication; Immunologic Pharmaceutical Corp.
79A. Bulone, V. 1998. Separation of horse dander allergen proteins by two-dimensional electrophoresis. Molecular characterisation and identification of Equ c 2.0101 and Equ c 2.0102 as lipocalin proteins. Eur J Biochem 253:202-211.
798. Swiss-Prot acc. P81216, P81217.
80. McDonald, B., M. C. Kuo, J. L. Ohman, and L. J. Rosenwasser. 1988. A 29 amino acid peptide derived from rat alpha 2 euglobulin triggers murine allergen specific human T cells (abst). J. Allergy Clin. Immunol. 83:251.
81. Clarke, A. J., P. M. Cissold, R. A. Shawi, P. Beattie, and J. Bishop. 1984. Structure of mouse urinary protein genes: differential splicing configurations in the 3'-non-coding region. EMBO J 3:1045-1052.
62. Longbottom, J. L 1983. Chracterization of allergens from the urines of experimental animals. McMillan Press, London, pup.525-529.
83. Laperche, Y., K. R. Lynch, K. P. Dolans, and P. Feigelsen. 1983. Tissue-specific control of alpha 2u globulin gene expression: constitutive synthesis in submaxillary gland. Cell 32:453-460.
83A. Aukrust L. Borch SM. 1979. Partial purification and characterization of two Cladosporium herbarum allergens. Int Arch Allergy Appl Immunol 60:68-79.
83B. Sward-Nordmo M, Paulsen BS, Wold JK. 1988. The glycoprotein allergen Ag-54 (Cla h 1f) from Cladosporium herbarum. Structural studies of the carbohydrate moiety. Int Arch Allergy AppI Immunol 85:288-294.
84. Shen, et al. J. Allergy Clin. Immunol. 103:S157, 1999.
84A. Crameri R. Epidemiology and molecular basis of the involvement of Aspergillus fumigatus in allergic diseases. Contrib. Microbiol. Vol. 2, Karger, Basel (in press).
84B. Shen, et al. (manuscript submitted), 1999
84C. Shen HD, Ling WL, Tan MF, Wang SR, Chou H, Han SIH. Vacuolar serine proteinase: A major allergen of Aspergillus fumigatus. 10th International Congress of Immunology, Abstract, 1998.
85. Kumar, A., L.V. Reddy. A. Sochanik, and V.P. Kurup. 1993. Isolation and characterization of a recombinant heat shock protein of Aspergillus fumigatus. J. Allergy Clin. Immunol. 91:1024-1030.
86. Teshima, R, H. Ikebuchi, J. Sawada, S. Miyachi, S. Kitani, M. Iwama, M. Iric, M. Ichinoe, and T. Terao. 1993. Isolation and characterization of a major allergenic component (gp55) of Aspergillus fumigatus. J. Allergy Clin. Immunol. 92:698-706.
86A. Shen HD, Lin WL, Tsai JJ, Liaw SF, Han SH. 1996. Allergenic components in three different species of Penicillium: crossreactivity among major allergens. Clin Exp Allergy 26:444-451.
86B. Shen, et al. Abstract; The XVIII Congress of the European Academy of Allergology and Clinical Immunology, Brussels, Belgium, 3-7 July 1999.
87. Shen HD, Liaw SF, Lin WL, Ro LH, Yang HL, Han SH. 1995. Molecular cloning of cDNA coding for the 68 kDa allergen of Penicillium notaturn using MoAbs. Clin Exp Allergy 25:350-356.
88. Shen, H.D., K.B. Choo, H.H. Lee, J.C. Hsich, and S.H. Han. 1991. The 40 kd allergen of Candida alhicans is an alcohol dehydrogencase: molecular cloning and immunological analysis using monoclonal antibodies. Clin. Exp. Allergy 21:675-681.
89. Shen, et al. Clin. Exp. Allergy (in press), 1999.
90. Woodfolk JA, Wheatley LM, Piyasena RV, Benjamin DC, Platts-Mills TA.1998. Trichophyton antigens associated with IgE antibodies and delayed type hypersensitivity. Sequence homology to two families of serine proteinases. J Biol Chem 273:29489-96.
91. Deuell, B., L.K. Arruda, M.L. Hayden, M.D. Chapman and T.A.E. Platts-Mills. 1991. Trichophyton tonsurans Allergen 1. J. Immunol. 147:96-101.
91A. Schmidt M, Zargari A, Holt P, Lindbom L, Hellman U, Whitley P, van der Ploeg I, Harfast B, Scheynius A. 1997. The complete cDNA sequence and expression of the first major allergenic protein of Malassezia furfur, Mal f I. Eur J Biochem 246:181-185. 91B. Homer WE, Reese G, Lehrer SB. 1995. Identification of the allergen Psi c 2 from the basidiomycete Psilocybe cubensis as a fungal cyclophilin. Int Arch Allergy Immunol 107:298-300.
92. Kuchler, K., M. Gmachl, M. J. Sippl, and G. Kreil. 1989. Analysis of the cDNA for phospholipase A2 from honey bee venom glands: The deduced amino acid sequence reveals homology to the corresponding vertebrate enzymes. Eur. J. Biochem. 14:249-254.
93. Gmachl, M., and G. Kreil. 1993. Bee venom hyaluronidase is homologous to a membrane protein of mammalian sperm. Proc. Natl. Acad. Sci. USA 90:3569-3573.
94. Habermann, E. 1972. Bee and wasp venoms. Science 177:314-322.
95. Jacobson, RS., and D.R Hoffman. 1993. Characterization of bumblebee venom allergens (abst). J. Allergy Clin. Immunol. 91:187.
96. Arruda LK, Vailes LD, Mann BJ, Shannon J, Fox JW, Vedvick TS, Hayden ML. Chapman MD. Molecular cloning of a major cockroach (Blattella germanica) allergen, Bla g 2. Sequence homology to the aspartic proteases. J Biol Chem 270:19563-19568, 1995.
97. Arruda LK, Vailes LD, Hayden ML, Benjamin DC, Chapman MD. Cloning or cockroach allergen, Bla g 4 identifies ligand binding proteins (or calycins) as a cause of IgE antibody responses. J Biol Chem 270:3119631201, 1995.
98. Arruda LK, Vailes LD, Benjamin DC, Chapman MD. Molecular cloning of German Cockroach (Blattella germanica) allergens. Int Arch Allergy Immunol 107:295-297, 1995.
98A. Wu CH, Lee MF, Liao SC. 1995. Isolation and preliminary characterization of cDNA encoding American cockroach allergens. J Allergy Clin Immunol 96: 352-9.
99. Mazur, G., X. Baur, and V. Liebers. 1990. Hypersensitivity to hemoglobins of the Diptera family Chironomidae: Structural and functional studies of their immunogenic/allergenic sites. Manog. Allergy 28:121-137.
100. Soldatova, L., L. Kochoumian, and T.P. King. 1993. Sequence similarity of a homet (D. maculata) venom allergen phospholipase Al with mammalian lipases. FEBS Letters 320:145-149.
101. Lu, G., L. Kochoumian and T.P. King. Whiteface hornet venom allergen hyaluronidase: cloning and its sequence similarity with other proteins (abst.). 1994. J. Allergy Clin. Immunol. 93:224.
102. Fang, K. S. F., M. Vitale, P. Fehlner, and T. P. King. 1988. cDNA cloning and primary structure of a white-faced homet venom allergen, antigen 5. Proc. Natl. Acad. Sci., USA 85:895-899.
103. King, T. P., D. C. Moran, D. F. Wang, L. Kochoumian, and B.T. Chait. 1990. Structural studies of a hornet venom allergen antigen 5, Dol m V and its sequence similarity with other proteins. Prot. Seq. Data Anal. 3:263-266.
104. Lu, G., M. Villalba, M.R Coscia, D.R. Hoffman, and T.P. King. 1993. Sequence analysis and antigen cross reactivity of a venom allergen antigen 5 from hornets, wasps and yellowjackets. J. Immunol. 150: 2823-2830.
105. King, T. P. and Lu, G. 1997. Unpublished data.
105A. King TP, Lu G, Gonzalez M, Qian N and Soldatova L. 1996. Yellow jacket venom allergens, hyaluronidase and phospholipase: sequence similarity and antigenic cross-reactivity with their hornet and wasp homologs and possible implications for clinical allergy. J. Allergy Clin. Immunol. 98:588-600.
106. Hoffman, D.R. 1993. Allergens in hymenaptera venom XXV: The amino acid sequences ofantigen 5 molecules and the structural basis of antigenic cross-reactivity. J. Allergy Clin. Immunol. 92:707-716.
107. Haffman, D.R 1992. Unpublished data.
108. Hoffman, D. R. 1993. The complete amino acid sequence of a yellowjacket venom phospholipase (abst). J. Allergy Clin. Immunol. 91:187.
109. Jacobson, R.S., D.R. Hoffman, and D.M. Kemeny. 1992. The cross-reactivity between bee and vespid hyaluronidases has a structural basis (abst). J. Allergy Clin. Immunol. 89:292.
110. Hoffman, D.R. 1993. Allergens in Hymenoptera venom XXIV: The amino acid sequences of imported fire ant venom allergens Sol i II, Sol i III, and Sol i IV. J. Allergy Clin. Immunol. 91:71-78.
111. Schmidt, M., R.B. Walker, D.R. Hoffman, and T.J. McConnell. 1993. Nucleotide sequence of cDNA encoding the fire ant venom protein Sol i II. FEBS Letters 319:138-140.
112. Elsayed S, Bennich H. The primary structure of Allergen M from cod. Scand J Immunol 3:683-686, 1974.
113. Elsayed S. Aas K, Sletten K, Johansson SGO. Tryptic cleavage of a homogeneous cod fish allergen and isolation of two active polypeptide fragments. Immunochemistry 9:647-661, 1972.
114. Hoffman, D. R. 1983. Immunochemical identification of the allergens in egg white. J. Allergy Clin. Immunol. 71:481-486.
115. Langeland, T. 1983. A clinical and immunological study of allergy to hen's egg white. IV. specific IgE antibodies to individual allergens in hen's egg white related to clinical and immunolgical parameters in egg-allergic patients. Allergy 38:493-500.
116. Daul, C.B., M. Slattery, J.E. Morgan, and S.B. Lehrer. 1993. Common crustacea allergens: identification of B cell epitopes with the shrimp specific monoclonal. antibodies. In: "Molecular Biology and Immunology of Allergens" (D. Kraft and A. Sehon, cds.). CRC Press, Bioca Raton. pp. 291-293.
117. K.N. Shanti, B.M. Martin, S. Nagpal, D.D. Metcalfe, P.V. Subba Rao. 1993. Identification of tropomyosin as the major shrimp allergen and characterization of its IgE-binding epitopes. J. Immunol. 15:5354-5363.
117A. M. Miyazawa, H. Fukamachi, Y. Inagaki, G. Reese, C.B. Daul, S.B. Lehrer, S. Inouye, M. Sakaguchi. 1996. Identification of the first major allergen of a squid (Todarodes pacificus). J. Allergy Clin. Immunol. 98:948-953.
117B A. Lopata et al. 1997. Characteristics of hypersensitivity reactions and identification of a uniques 49 kDa IgE binding protein (Hal-m-I) in Abalone (Haliotis midae). J.Allergy Clin. Immunol. Submitted
118. Monsalve, R.I., M.A. Gonzalez de la Pena, L Menendez-Arias, C. Lopez-Otin, M. Villalba, and R. Rodriguez. 1993. Characterization of a new mustard allergen, Bra j IE. Detection of an allergenic epitope. Biochem. J. 293:625-632.
119. Mena, M., R. Sanchez-Monge, L. Gomez. G. Salcedo, and P. Carbonero. 1992. A major barley allergen associated with baker's asthma disease is a glycosylated monomeric inhibitor of insect alpha-amylase: cDNA cloning and chromosomal location of the gene. Plant Molec. Biol. 20:451-45B.
120. Menendez-Arias, L., I. Moneo, J. Dominguez, and R. Rodriguez. 1988. Primary structure of the major allergen of yellow mustard (Sinapis alba L.) seed, Sin a I. Eur. J. Biochem. 177:159-166.
121. Gonzalez R, Varela J, Carreira J, Polo F. Soybean hydrophobic protein and soybean hull allergy. Lancet 346:48-49, 1995.
122. Christie, J. F., B. Dunbar, I. Davidson, and M. W. Kennedy. 1990. N-terminal amino acid sequence identity between a major allergen of Ascaris lumbricoides and Ascaris suum and MHC-restricted IgE responses to it. Immunology 69:596-602.
123. Czuppon AB, Chen Z, Rennert S, Engelke T, Meyer HE, Heber M, Baur X. The rubber elongation factor of rubber trees (Hevea brasiliensis) is the major allergen in latex. J Allergy Clin Immunol 92:690-697, 1993.
124. Attanayaka DPSTG, Kekwick RGO, Franklin FCH. 1991. Molecular cloning and nuelcotide sequencing of the rubber elongation factor gene from hevea brasiliensis. Plant Mol Biol 16:1079-1081.
125. Chye ML, Cheung KY. 1995. ( 1,3-glucanase is highly expressed in Laticifers of Hevea brasiliensis. Plant Mol Biol 26:397-402.
126. Alenius H, Palosuo T, Kelly K, Kurup V, Reunala T, Makinen-Kiljunen S, Turjanmaa K Fink J. 1993. IgE reactivity to 14-kD and 27-kD natural rubber proteins in Latex-allergic children with Spina bifida and other congenital anomalies. Int Arch Allergy Immunol 102:61-66.
127. Yeang HY, Cheong KF, Sunderasan E, Hamzah S, Chew NP, Hemid S, Hamilton RG, Cardosa MJ. 1996. The 14.6 kD (REF, Hev b 1) and 24 kD (Hev b 3) rubber particle proteins are recognized by IgE from Spina Bifida patients with Latex allergy. J Allerg Clin Immunol in press.
128. Sunderasan E, Hamzah S, Hamid S, Ward MA, Yeang HY, Cardosa MJ. 1995. Latex B-serum (-1,3-glucanase (Hev b 2) and a component of the microhelix (Hev b 4) are major Latex allergens. J nat Rubb Res 10:82-99.

## Claims

1. Composition comprising:
a) at least one allergen; and
b) a non-ADP ribosylating toxin or subunit thereof selected from a B subunit of an ADP ribosylating AB exotoxin and an aldehyde-treated AB exotoxin, for use as a medicament to be transdermally administered to a mammal suffering from an IgE-mediated hypersensitivity allergic reaction wherein the amounts of a) and b) together are effective to suppress said allergic reaction,
said composition being essentially devoid of ADP-ribosylating activity.

2. Composition according to claim 1, wherein the B subunit of an ADP ribosylating AB exotoxin is selected from cholera toxin B subunit (CTB), *E. coli* heat-labile toxin B subunit (LTB), mutants and derivatives thereof, provided that said mutants and derivatives react with polyclonal antiserum to CTB or LTB and have essentially the same biological effects as CTB or LTB when administered transdermally.

3. Composition according to claim 1 or 2, wherein the allergen and the B subunit of an ADP ribosylating AB exotoxin are chemically linked.

4. Composition according to claim 1 or 2, wherein the allergen and the B subunit of an ADP ribosylating AB exotoxin are genetically fused.

5. Composition according to claim 1 or 2, wherein the allergen and the B subunit of an ADP ribosylating AB exotoxin are mixed together.

6. Composition according to any of claims 1 to 5, wherein the medicament is in the form of a patch.

7. Composition according to any of claims 1 to 6, wherein the composition comprises a plurality of allergens.

8. Composition according to any of claims 1 to 7, wherein the B subunit of an ADP ribosylating AB exotoxin is CTB.

9. Composition according to any of claims 1 to 8, wherein the hypersensitivity reaction comprises an immediate hypersensitivity reaction.

10. Composition according to claim 1, wherein the allergen is a derivative, analog or mutant of an allergen, provided that said derivative, analog or mutant reacts with polyclonal antiserum to said allergen and has essentially the same biological effects as said allergen when administered transdermally.

11. Composition according to claim 10, wherein the derivative comprise at least one epitope capable of interacting with specific IgE antibodies.

12. Composition according to claim 1, wherein the composition comprises an aldehyde-treated AB exotoxin.

13. Composition according to claim 12, wherein the composition is administered in a formulation containing an aldehyde.

14. Composition according to claim 12, wherein the aldehyde-treated AB exotoxin is cholera toxin.

## Patentansprüche

1. Zusammensetzung, umfassend:
a) mindestens ein Allergen; und
b) ein nicht-ADP ribosylierendes Toxin oder eine Untereinheit davon, das die aus einer B Untereinheit eines ADP ribosylierenden AB Exotoxins und einem mit Aldehyd behandelten AB Exotoxin ausgewählt ist, zur Verwendung als Medikament zur transkutanen Verabreichung bei einem Säugetier, welches an einer IgE-vermittelten, allergischen Hypersensitivitätsreaktion leidet, wobei die Mengen an a) und b) zusammen effektiv sind, um besagte allergische Reaktion zu unterdrücken, wobei besagte Zusammensetzung im wesentlichen frei von ADP ribosylierender Aktivität ist.

2. Zusammensetzung gemäß Anspruch 1, wobei die B Untereinheit eines ADP ribosylierenden AB Exotoxins ausgewählt ist aus einer Choleratoxin B Untereinheit (CTB), einer *E. coli* hitzelabiler Toxin B Untereinheit (LTB), Mutanten oder Derivate davon, mit der Maßgabe, dass besagte Mutanten und Derivate mit polyklonalem Antiserum zu CTB oder LTB reagieren und im wesentlichen die gleichen biologischen Effekte wie CTB oder LTB besitzen, wenn sie transkutan verabreicht werden.

3. Zusammensetzung gemäß Anspruch 1 oder 2, wobei das Allergen und die B Untereinheit eines ADP ribosylierenden AB Exotoxins chemisch verknüpft sind.

4. Zusammensetzung gemäß Anspruch 1 oder 2, wobei das Allergen und die B Untereinheit eines ADP ribosylierenden AB Exotoxins genetisch fusioniert sind.

5. Zusammensetzung gemäß Anspruch 1 oder 2, wobei das Allergen und die B Untereinheit eines ADP ribosylierenden AB Exotoxins miteinander gemischt sind.

6. Zusammensetzung gemäß einem der Ansprüche 1 bis 5, wobei das Medikament in der Form eines Pflasters vorliegt.

7. Zusammensetzung gemäß einem der Ansprüche 1 bis 6, wobei die Zusammensetzung eine Vielzahl von Allergenen umfasst.

8. Zusammensetzung gemäß einem der Ansprüche 1 bis 7, wobei die B Untereinheit eines ADP ribosylierenden AB Exotoxins CTB ist.

9. Zusammensetzung gemäß einem der Ansprüche 1 bis 8, wobei die Hypersensitivitätsreaktion eine unmittelbare Hypersensitivitätsreaktion umfasst.

10. Zusammensetzung gemäß Anspruch 1, wobei as Allergen ein Derivat, Analog oder Mutant eines Allergens ist, mit der Maßgabe, dass besagtes Derivat, Analog oder besagter Mutant mit polyklonalem Antiserum zu besagtem Allergen reagiert und im wesentlichen die gleichen biologischen Effekte wie besagtes Allergen besitzt, wenn es transkutan verabreicht wird.

11. Zusammensetzung gemäß Anspruch 10, wobei das Derivat mindestens ein Epitop umfasst, das in der Lage ist mit spezifischen IgE Antikörpern zu interagieren.

12. Zusammensetzung gemäß Anspruch 1, wobei die Zusammensetzung ein mit Aldehyd behandeltes AB Exotoxin umfasst.

13. Zusammensetzung gemäß Anspruch 12, wobei die Zusammensetzung in einer Formulierung verabreicht wird, die ein Aldehyd beinhaltet.

14. Zusammensetzung gemäß Anspruch 12, wobei das mit Aldehyd behandelte AB Exotoxin ein Choleratoxin ist.

## Revendications

1. Composition comprenant :
a) au moins un allergène ; et
b) une toxine non ADP-ribosylante ou une sous-unité de celle-ci choisie parmi une sous-unité B d'une exotoxine AB ADP-ribosylante et d'une exotoxine AB traitée par un aldéhyde, pour une utilisation en tant que médicament à administrer par voie transdermique à un mammifère souffrant d'une réaction allergique d'hypersensibilité médiée par les IgE où les quantités de a) et b) ensemble sont efficaces pour supprimer ladite réaction allergique, ladite composition étant pratiquement dépourvue d'activité ADP-ribosylante.

2. Composition selon la revendication 1, dans laquelle la sous-unité B d'une exotoxine AB ADP-ribosylante est choisie parmi la sous-unité B de la toxine cholérique (CTB), la sous-unité B de la toxine thermolabile d'*E. coli* (LTB), des mutants et des dérivés de celles-ci, sous réserve que lesdits mutants et dérivés réagissent avec un antisérum polyclonal dirigé contre la CTB ou la LTB et aient pratiquement les mêmes effets biologiques que la CTB ou la LTB lorsqu'ils sont administrés par voie transdermique.

3. Composition selon la revendication 1 ou 2, dans laquelle l'allergène et la sous-unité B d'une exotoxine AB ADP-ribosylante sont liés chimiquement.

4. Composition selon la revendication 1 ou 2, dans laquelle l'allergène et la sous-unité B d'une exotoxine AB ADP-ribosylante sont fusionnés génétiquement.

5. Composition selon la revendication 1 ou 2, dans laquelle l'allergène et la sous-unité B d'une exotoxine AB ADP-ribosylante sont mélangés ensemble.

6. Composition selon l'une quelconque des revendications 1 à 5, où le médicament est sous la forme d'un patch.

7. Composition selon l'une quelconque des revendications 1 à 6, où la composition comprend une pluralité d'allergènes.

8. Composition selon l'une quelconque des revendications 1 à 7, dans laquelle la sous-unité B d'une exotoxine AB ADP-ribosylante est la CTB.

9. Composition selon l'une quelconque des revendications 1 à 8, où la réaction d'hypersensibilité comprend une réaction d'hypersensibilité immédiate.

10. Composition selon la revendication 1, dans laquelle l'allergène est un dérivé, un analogue ou un mutant d'un allergène, sous réserve que ledit dérivé, analogue ou mutant réagisse avec un antisérum polyclonal dirigé contre ledit allergène et ait pratiquement les mêmes effets biologiques que ledit allergène lorsqu'il est administré par voie transdermique.

11. Composition selon la revendication 10, dans laquelle le dérivé comprend au moins un épitope capable d'interagir avec des anticorps IgE spécifiques.

12. Composition selon la revendication 1, où la composition comprend une exotoxine AB traitée par un aldéhyde.

13. Composition selon la revendication 12, où la composition est administrée dans une formulation contenant un aldéhyde.

14. Composition selon la revendication 12, dans laquelle l'exotoxine AB traitée par un aldéhyde est la toxine cholérique.
